# EUROPÄISCHE PATENTSCHRIFT

(11) **EP 2 661 732 B1**
(45) Veröffentlichungstag und Bekanntmachung des Hinweises auf die Patenterteilung: **29.07.2015**
(21) Anmeldenummer: 11794652.5
(22) Anmeldetag: 12.12.2011
(51) Int. Cl.: G06T 7/00, A61C 13/00, G06K 9/62

(54) **VERFAHREN UND ZAHNRESTAURATIONSERMITTLUNGSSYSTEM ZUR ERMITTLUNG VON ZAHNRESTAURATIONEN**
METHOD AND TOOTH RESTORATION DETERMINATION SYSTEM FOR DETERMINING TOOTH RESTORATIONS
PROCÉDÉ ET SYSTÈME D'IDENTIFICATION DE RESTAURATIONS DENTAIRES, DESTINÉS À IDENTIFIER DES RESTAURATIONS DENTAIRES

(30) Priorität: 07.01.2011 DE 102011008074
(43) Veröffentlichungstag der Anmeldung: 13.11.2013
(73) Patentinhaber: Straumann Holding AG, 4002 Basel (CH)
(72) Erfinder: TANK, Martin, 69115 Heidelberg (DE)
(74) Vertreter: Beckord, Klaus
(86) Internationale Anmeldenummer: PCT/EP2011/006257
(87) Internationale Veröffentlichungsnummer: WO 2012/092946

(56) Entgegenhaltungen:
- WO-A1-2004/081853
- WO-A2-2004/044787
- DE-A1-102007 033 998

## Beschreibung

Die Erfindung betrifft ein Verfahren zur Ermittlung von virtuellen Zahnrestaurationen auf der Basis von Scandaten oraler Strukturen, welches in dentalen CAD/CAM-Systemen eingesetzt werden kann. Solche Systeme sind mittlerweile in der Zahnmedizin, Zahntechnik und Kieferorthopädie fest etabliert, wobei man in den exemplarischen Patentschriften US 5217375A, US 7708560 B2, US 7581953 B2, EP 06 34 150 A1, EP 09 13 130 A2, DE 10 2005 033 738 A1 und WO 0239056 A1 Beschreibungen solcher Systeme findet. Unter oralen Strukturen werden im Rahmen der vorliegenden Erfindung Zähne mit ihren angrenzenden anatomischen Strukturen wie Zahnfleisch, Kieferknochen, Nervenbahnen etc. aber auch nichtnatürliche Strukturen wie Implantate, Abutments, sonstige Verankerungssysteme, statische sowie funktionelle Bissregistrate etc. verstanden. Außerdem betrifft die Erfindung ein Verfahren zur Erzeugung einer Modelldatenbank zur Verwendung in einem derartigen Verfahren sowie ein Zahnrestaurationsermittlungssystem zur Ermittlung von virtuellen Zahnrestaurationen. Weiterhin betrifft die Erfindung ein Verfahren zur Herstellung oder Auswahl eines Zahnrestaurationsteils.

Auf dem Gebiet der digitalen dentalen Technologie werden Scandaten oraler Strukturen vor allem optisch oder radiologisch bestimmt. Weit verbreitet und kostengünstig sind optische Scanner, welche intraoral Oberflächenstrukturen direkt oder extraoral Abformungen von oralen Oberflächenstrukturen dreidimensional vermessen. Man erhält in der Regel triangulierte Oberflächendaten, die bei offenen Systemen im STL-Format abgespeichert werden können. Mittels radiologischer Scanner wie Digitalen Volumentomographen (DVT) oder Computertomographen (CT) werden Volumendatensätze oraler Strukturen durch Verwendung von Röntgenstrahlung erzeugt. Aus diesen Volumendatensätzen lassen sich auch Oberflächenstrukturen durch die Anwendung von Schwellenwertverfahren extrahieren, bei denen die Intensitätswerte (gemessen in Hounsfield-Einheiten) der einzelnen Scanelemente daraufhin analysiert werden, ob sie einen bestimmten Schwellenwert überschreiten. Radiologisch dichte Strukturen wie Zähne lassen sich so selektieren und als triangulierte Oberflächendaten verwenden. Etwas aufwändiger sind die Berechnungen bei MR-Scandaten, wo vorzugsweise Konturanalyseverfahren Anwendung finden, die auf Basis von Gradienten benachbarter Scanelemente arbeiten. Durch die aufgeführten Analyseverfahren kann man für alle Modalitäten (Bildaufnahmegeräte) triangulierte Oberflächendaten gewünschter Grenzflächen in den Scandaten erhalten. Im Rahmen der vorliegenden Erfindung können die Scandaten z.B. alle zuvor erwähnten Datenarten, unabhängig von der Art ihrer Gewinnung, aber auch weitere algorithmisch und/oder interaktiv bestimmte Strukturen wie Präparations-, Segmentierungslinien und anatomische Landmarken sowie gewünschte Kontaktpunkte für die virtuellen Zahnrestaurationen etc. umfassen.

In der Regel werden aufgrund der nötigen hohen Präzision bei der Ermittlung bzw. Berechnung von virtuellen Zahnrestaurationen optische Scandaten verwendet. Unter Zahnrestaurationsteilen werden im Rahmen der vorliegenden Erfindung jegliche Art von herstellbaren Objekten zur Versorgung von Zahndefekten zusammengefasst. Beispielhaft seien hier Inlays, Onlays, Teilkronen, Kronen, Teleskopkronen, Brücken, Veneers, Implantataufbauten, Teilprothesen und Prothesen genannt. Es sei hier nochmals betont, dass im Rahmen der vorliegenden Erfindung auch Zahnersatzteile der Einfachheit halber unter dem Begriff Zahnrestaurationsteile fallen. Unter dem Begriff der "virtuellen Zahnrestauration" (im Folgenden auch kürzer als "Zahnrestauration" bezeichnet) sind entsprechende elektronische Zahnrestaurationsdarstellungen, d.h. digitale dreidimensionale Repräsentierungen solcher Zahnrestaurationsteile zu verstehen. Zur Herstellung eines Zahnrestaurationsteils wird dann beispielsweise ein CAD/CAM-Datensatz der dazugehörigen virtuellen Zahnrestauration an eine Herstellungsmaschine übermittelt.

Bei der Versorgung von Zahndefekten findet in der Regel eine Präparation der Zähne statt, d.h. es werden Karies, altes Füllungsmaterial oder defekte Zahnanteile entfernt. Übrig bleibt für jeden Zahn die Restzahnsubstanz, deren Oberfläche sich in einen präparierten (Kavität) und unpräparierten Anteil aufteilt. Die Grenzlinie von unpräparierter Zahnoberfläche zu präparierter Zahnoberfläche wird dabei als Präparationslinie bezeichnet. Ergänzend zu den nur bei präparierten Zähnen vorhandenen Präparationslinien besitzt jeder Zahn zumindest eine Segmentierungslinie, welche einen Zahn von extradentalen Strukturen wie beispielsweise dem Zahnfleisch und/oder den Nachbarzähnen und/oder dem Kieferknochen trennt. Aus diesen Definitionen ergibt sich, dass die Segmentierungs- und/oder Präparationslinien unpräparierte Zahnoberflächen begrenzen. Diese Grenzlinien können im Rahmen der Erfindung interaktiv in den Scandaten eingezeichnet werden, wobei in der Regel eine algorithmische Unterstützung bei deren Bestimmung stattfindet, welche auf der Analyse von Flächenkrümmungen der Scandaten basiert.

Aufgrund der komplizierten Struktur der Scandaten und der Vielzahl der einzuhaltenden funktional-ästhetischen Kriterien (Anpassung an Gegenbezahnung unter evtl. Berücksichtigung der Kieferbewegung, Anpassung an Nachbarbezahnung unter Berücksichtigung von Kontaktpunkten, Anpassung an die Präparationslinien, um einen optimalen Randschluss zu erreichen, Einhaltung minimaler Materialstärken, um eine gute mechanische Stabilität zu erzielen, Berücksichtigung gewünschter Zahnformen im Frontzahnbereich, etc.) ist die interaktive computergestützte Erstellung von Zahnrestaurationen mit Hilfe einer grafischen Benutzeroberfläche oft schwierig durchzuführen und mit einem hohen Zeitaufwand verbunden. Eine Verbesserung stellt die Anwendung von modellbasierten Verfahren dar, bei denen morphologisches Vorwissen über die anatomischen Variationen der natürlichen Zahnformen in die Berechnung von Zahnrestaurationen einfließt und die funktional-ästhetischen Kriterien in Form von Optimierungskriterien bei der Anpassung der Zahnmodelle an die Scandaten berücksichtigt werden.

Aufgrund der wirtschaftlichen Bedeutung der prothetischen Zahnversorgung gibt es eine Reihe von modellbasierten Verfahren zur Ermittlung bzw. Berechnung von Zahnrestaurationen. Bei computergestützten CAD/CAM-Verfahren ist die grundsätzliche Vorgehensweise bei allen Verfahren ähnlich. Zuerst werden dreidimensionale digitale Zahnmodelle an die Scandaten angepasst, wobei dieser Vorgang üblicherweise auch als "Individualisierung" von Zahnmodellen bezeichnet wird. Danach findet eine Berechnung der Zahnrestaurationen auf Basis der individualisierten Zahnmodelle und Scandaten statt. Abschließend werden entsprechend den Zahnrestaurationen die passenden Zahnrestaurationsteile in einer Maschine gefertigt werden.

In der DE 102 52 298 B3 wird ein Verfahren beschrieben, bei dem die verwendete Modelldatenbank auf einer Hauptkomponentenanalyse einer größeren Menge von Probanden-Zahnscans basiert. Die Parametrisierung der Zahnmodelle besteht dabei aus den Linearfaktoren der wichtigsten Hauptkomponenten. Eine Beschreibung einer besonderen Suchstrategie für die Verwendung von geometrisch nicht deformierbaren Zahnmodellen findet sich in dieser Patentschrift nicht. Des Weiteren wird die Individualisierung von Zahnmodellen bei diesem Verfahren für einen einzigen gewünschten Zahntyp durchgeführt und nicht für Gruppen von gekoppelten Zahnmodellen unterschiedlicher Zahntypen. Dabei entspricht der Zahntyp in dieser Patentschrift in der Regel einer Zahnnummer, kann aber auch Mengen von Zahnmodellen nach anderen Kriterien (Alter, morphologische Eigenschaften, etc.) beschreiben. Für den Fall, dass sich die Zahnmodelle einer Zahnnummer in mehrere Zahntypen aufteilen, wird in dieser Schrift keine effektive Suchstrategie zur automatischen Bestimmung des zu verwendenden Zahntyps beschrieben. Insbesondere ist bei der Verwendung von Zahntypen, die nach morphologischen Kriterien definiert werden, offen, nach welchem automatisierten Algorithmus die Zuordnung der Zahnmodelle zu den Zahntypen stattfindet.

In der US 2006/0183082 wird ein weiteres Verfahren zur Berechnung von Zahnrestaurationen vorgeschlagen, bei dem die Zahnmodelle einer Modelldatenbank nach Typ, Alter, Geschlecht und/oder Zugehörigkeit zu einer ethnischen Gruppe sortiert sein können und jedes Zahnmodell eine spezielle geschlossene Kantenlinie am Rand der Okklusionsfläche besitzt. Eine Beschreibung, wie aus solch einer Modelldatenbank automatisch und effektiv optimale Zahnmodelle zur Bestimmung von Zahnrestaurationen bestimmt werden, findet sich in dieser Schrift nicht. In der beschriebenen Modelldatenbank sind auch keine Informationen über zulässige geometrische Deformationen der Zahnmodelle enthalten. Der Schwerpunkt dieser Schrift liegt eher auf der Konstruktion von Zahnrestaurationen nach erfolgter Individualisierung. Dazu werden Kantenlinien individualisierter Zahnmodelle nach speziellen geometrischen Regeln mit den dazugehörigen Präparationslinien der Scandaten verbunden, um die noch fehlenden Seitenflächen der Zahnrestaurationen zu konstruieren.

Durch die Patentschriften DE 20 2007 014 550 U1, DE 20 2005 020 715 U1, DE 10 2006 043 284 A1, DE 10 2004 038 136 A1, DE 196 42 247 C1, DE 199 23 978 A1, DE 198 38 239 A1, EP 06 43 948 A1, US 2009/0148816 A1 und US 7433810 B2 sind Berechnungsverfahren bekannt, bei denen aufgrund vielfältiger Gründe nicht natürliche Formen von Zahnrestaurationen entstehen und/oder bei denen die ermittelten Zahnrestaurationen funktional-ästhetische Defizite aufweisen können. Dies liegt mitunter an der fehlenden Automatisierung der Verfahren, da zum Teil erhebliche Bediener-Interaktionen bei der Optimierung der Zahnmodelle stattfinden. Insbesondere sind interaktiv gesteuerte geometrische Deformationen größeren Umfangs der Zahnmodelle (Positionierung anatomischer Landmarken, Herstellung gewünschter Kontaktpunkte, etc.) kritisch zu betrachten, da sie in hohem Maß subjektiv und nicht reproduzierbar sind.

Es ist eine Aufgabe der vorliegenden Erfindung, ein modellbasiertes Verfahren und Zahnrestaurationsermittlungssystem zur einfachen und sicheren Ermittlung von virtuellen Zahnrestaurationen gewünschter Zahntypen, unter Einhaltung funktional-ästhetischer Kriterien zu beschreiben, wobei die Ermittlung mit möglichst geringer Bediener-Interaktion und geringem Zeitaufwand zufriedenstellend möglich ist. Dabei soll das Verfahren sowohl geometrisch deformierbare Zahnmodelle als auch geometrisch nicht deformierbare Zahnmodelle verwenden können.

Diese Aufgabe wird durch ein Verfahren gemäß dem Patentanspruch 1, ein Verfahren zur Erzeugung einer Modelldatenbank gemäß Patentanspruch 11, sowie durch ein Zahnrestaurationsermittlungssystem gemäß Patentanspruch 15 gelöst.

Im Rahmen der vorliegenden Erfindung werden die grundlegenden Begriffe Zahntyp und Zahnmodell sehr allgemein verstanden. Ein Zahntyp wird im Rahmen der Erfindung als eine sehr allgemeine Gruppierungsmöglichkeit von Zähnen definiert. Beispielsweise kann eine Gruppierung nach der Zahnnummer und/oder dem Alter und/oder der Abrasion und/oder der Volkszugehörigkeit und/oder dem Geschlecht und/oder nach morphologischen Besonderheiten (Wurzelanzahl, Höckeranzahl, etc.) erfolgen. Die Gruppierung kann aber auch durch die Zugehörigkeit zu den Frontzähnen, Eckzähnen, Prämolaren, Molaren oder zum Ober- bzw. Unterkiefer durchgeführt werden. Möglich ist auch eine Gruppierung von Zähnen unterschiedlicher Zahnnummern zu einem komplexen Zahntyp. An dieser Stelle sei betont, dass in der Praxis die Gruppierung über die Zahnnummer bevorzugt ist, d.h. Zahntyp und Zahnnummer können dann synonym verwendet werden. Ebenso sehr allgemein wird der Begriff Zahnmodell verwendet. Ein Zahnmodell kann dabei aus mehreren Modell-Teilen bestehen, beispielsweise aus anatomischen Strukturen wie Zahnoberflächen, Zahnfleisch, Kieferknochen, Nervenstrukturen, etc., aber auch aus nichtnatürlichen Strukturen wie Implantaten, Abutments, sonstigen Verankerungssystemen, etc.. Darüber hinaus kann ein Zahnmodell auch Präparations-, Segmentierungslinien, anatomische Landmarken, Zahnachsen, Richtungsbezeichnungen, Oberflächenregionen und weitere charakteristische geometrische Strukturen enthalten. Wichtig ist, dass zumindest diejenigen Anteile der Zähne modelliert werden, welche zur Ermittlung von virtuellen Zahnrestaurationen nötig sind. Die anderen Modell-Teile dienen vorzugsweise vor allem der Stabilisierung des Individualisierungsvorgangs, da diese an korrespondierende Strukturen in den Scandaten angepasst werden können.

Zur Ermittlung von virtuellen Zahnrestaurationen werden erfindungsgemäß parametrisierte Zahnmodelle aus einer speziell aufgebauten Modelldatenbank verwendet, bei der für jeden Zahntyp eine Anzahl von Zahnmodellen vorhanden ist. Die Menge aller Zahnmodelle eines Zahntyps wird im Folgenden auch als Modellklasse bezeichnet, wobei sich die Zahnmodelle einer Modellklasse in ihrer räumlichen Auflösung und ihrem topologischen Aufbau etc. unterscheiden können (z.B. unterschiedliche Triangulierung, unterschiedliche Modell-Teile, unterschiedliche anatomische Landmarken, etc.). Die Parametrisierung erfolgt durch Modellparameter, welche Lage- und/oder Formparameter umfassen. Dabei beschreiben die sechs Lageparameter eines Zahnmodells die räumliche Position und Orientierung eines Zahnmodells in den Scandaten. Anatomisch sinnvolle Formvariationen jedes einzelnen Zahnmodells können dabei optional durch parametrisierte geometrische Transformationen erzeugt werden, die sich von Zahnmodell zu Zahnmodell unterscheiden können und in der Modelldatenbank hinterlegt sind. D.h., die Anzahl der Formparameter kann von Zahnmodell zu Zahnmodell variieren. Darüber hinaus können die Modellparameter neben den Lage- und/oder Formparametern weitere Parameter umfassen, die z.B. Regionen und/oder Grenzlinien auf den Oberflächen der Zahnmodelle und/oder Materialeigenschaften parametrisieren.

Erfindungsgemäß wird für jeden gewünschten Zahntyp ein optimales Zahnmodell aus einer Modelldatenbank bestimmt, welches einen maximalen Qualitätswert der Individualisierung aufweist. Unter Individualisierung wird die Anpassung von Zahnmodellen an Scandaten verstanden, wobei Optimierungskriterien, wie z.B. die Anpassung von Zahnmodellen an die Zähne und/oder Restzahnsubstanzen, möglichst gut eingehalten werden sollen. Der Qualitätswert einer Individualisierung beschreibt dann quantitativ, wie gut die Optimierungskriterien eingehalten wurden.

Die Bestimmung eines optimalen Zahnmodells aus der Modelldatenbank erfolgt erfindungsgemäß durch ein iteratives Verfahren. Dabei wird zunächst zumindest ein Start-Zahnmodell eines gewünschten Zahntyps aus der Modelldatenbank ausgewählt und dann beginnend mit diesem Start-Zahnmodell bei jedem Iterationsschritt ein Zahnmodell hinsichtlich eines Qualitätswerts getestet. Dazu wird das aktuell zu testende Zahnmodell durch Variation von Modellparametern individualisiert und ein Qualitätswert für die Individualisierung berechnet. Anschließend oder parallel wird zumindest ein mit dem zu testenden Zahnmodell verlinktes Zahnmodell ebenfalls individualisiert und ein Qualitätswert berechnet. Auf Basis der berechneten Qualitätswerte findet dann gegebenenfalls, z.B. wenn sich durch den Vergleich der Qualitätswerte ergibt, dass ein anderes Zahnmodell besser geeignet als das aktuell zu testende Zahnmodell (bzw. im ersten Iterationsschritt das Start-Zahnmodell) ist, eine Auswahl eines neu zu testenden Zahnmodells des gewünschten Zahntyps für den nächsten Iterationsschritt statt. Über den Abbruch der Iteration entscheidet die Erfüllung eines Qualitätskriteriums z.B. am Ende eines Iterationsschritts.

Abschließend wird aus den optimalen Zahnmodellen und Scandaten zumindest eine Zahnrestauration bestimmt. Die entsprechenden Verfahren sind dem Fachmann bekannt. Dazu werden prinzipiell die Präparationslinien in geeigneter Weise möglichst stetig und glatt mit den dazugehörigen Zahnmodellen verbunden und dann die Kavitäten der präparierten Zähne als untere Begrenzungen hinzugefügt. Als Ergebnis erhält man für die Zahnrestaurationen dreidimensionale Modelle von Schleifkörpern, die maschinell hergestellt werden können. Man kann aber auch die Zahnrestaurationen mehrteilig gestalten, indem zuerst ein Trägergerüst und danach eine Oberkonstruktion mit den Kauflächen bestimmt wird. Insbesondere zur Prothesenherstellung ist außerdem die Herstellung einer Negativform der ermittelten Zahnrestaurationen vorteilhaft. Mit ihrer Hilfe lassen sich die Zahnrestaurationsteile im Rahmen der Prothesenherstellung dreidimensional aufstellen.

Das erfindungsgemäße Verfahren erlaubt es die Zahnmodelle mit praktikablem Rechenaufwand und möglichst automatisiert einzusetzen. Insbesondere kann das Verfahren aufgrund der besonders effektiven iterativen Suchstrategie mittels untereinander verlinkter Zahnmodelle eines Zahntyps zur Auffindung von optimalen Zahnmodellen auch mit geometrisch nicht deformierbaren Zahnmodellen genutzt werden. Dies ist vor allem hilfreich, wenn eine begrenzte Menge von günstig herzustellenden vorkonfektionierten künstlichen Zähnen (Prothesenzähne, Zahnrohlinge, etc.) für die prothetische Zahnversorgung verwendet werden soll. Ein Beispiel für diesen Ansatz ist die Herstellung von Vollprothesen. Bei dem dazugehörigen computergestützten Berechnungsverfahren sollen die den künstlichen Zähnen entsprechenden Zahnmodelle keinen geometrischen Deformationen unterworfen werden. Dabei kann die Anzahl der zur Verfügung stehenden Zahnmodelle erheblich sein, so dass für diesen Fall effektive Suchkriterien zur Bestimmung von optimalen Zahnmodellen zur Bestimmung von Zahnrestaurationen besonders wichtig sind. Solche prothetischen Zahnversorgungen sind insoweit interessant, da eine Herstellung von individuellen Zahnrestaurationen mit erheblichen Kosten verbunden ist.

Die abhängigen Ansprüche sowie die nachfolgende Beschreibung enthalten jeweils besonders vorteilhafte Ausgestaltungen und Weiterbildungen der Erfindung, wobei insbesondere das erfindungsgemäße Zahnrestaurationsermittlungssystem auch analog zu den Merkmalen der abhängigen Verfahrensansprüche weitergebildet sein kann. Zudem können im Rahmen der Erfindung auch die verschiedenen Merkmale unterschiedlicher Ausführungsbeispiele zu neuen Ausführungsbeispielen kombiniert werden.

Bei einer bevorzugten Weiterbildung des erfindungsgemäßen Verfahrens wird zunächst für jeden gewünschten Zahntyp einer Gruppe von Zahntypen zumindest ein Start-Zahnmodell aus der Modelldatenbank ausgewählt und dann beginnend mit diesen Start-Zahnmodellen bei jedem Iterationsschritt entsprechend eine Gruppe von Zahnmodellen hinsichtlich eines Qualitätswerts (Gruppenqualitätswert) getestet. Dazu wird die aktuell zu testende Gruppe von Zahnmodellen durch Variation von Modellparametern individualisiert und ein Qualitätswert für die Individualisierung berechnet. Anschließend oder parallel wird zumindest eine weitere Gruppe von Zahnmodellen (entsprechend der Gruppe der Zahntypen) ebenfalls individualisiert und ein weiterer Qualitätswert berechnet. Diese weitere Gruppe ergibt sich durch Variation der aktuell zu testenden Gruppe derart, dass zumindest ein Zahnmodell der aktuell zu testenden Gruppe durch ein mit ihm verlinktes Zahnmodell ersetzt wird. Auf Basis der berechneten Qualitätswerte findet dann gegebenenfalls eine Auswahl einer neuen zu testenden Gruppe von Zahnmodellen der gewünschten Gruppe von Zahntypen statt. Über den Abbruch der Iteration entscheidet auch hier wieder die Erfüllung eines Qualitätskriteriums, z.B. am Ende eines Iterationsschritts. Dieses Verfahren erlaubt es also, neben einzelnen Zahnmodellen auch Gruppen von Zahnmodellen optimal an die Scandaten anzupassen, was insbesondere für Kronen- und Brückenkonstruktionen von Vorteil ist.

Vorzugsweise erfolgt die Individualisierung von Zahnmodellen durch die Definition und Lösung einer Optimierungsaufgabe. Zur Lösung der Optimierungsaufgabe werden Modellparameter solange variiert, bis der Optimierungswert minimal oder ein Abbruchkriterium erfüllt ist. Dabei kann sich der Optimierungswert der Optimierungsaufgabe besonders bevorzugt aus einer Anzahl von Optimierungsteilwerten zusammensetzen, die jeweils bestimmten gewünschten Optimierungskriterien entsprechen.

Bevorzugte Optimierungsteilwerte beschreiben die Anpassung der Zahnmodelle an die Zähne und/oder an die Restzahnsubstanzen, d.h. die modellierten Zahnoberflächen der Zahnmodelle sollen mit den dazugehörigen unpräparierten Zahnoberflächen der Scandaten möglichst gut zur Deckung gebracht werden. Weitere bevorzugte Optimierungsteilwerte beschreiben die Anpassung der Zahnmodelle an die Gegenbezahnung und/oder an statische Bissregistrate und/oder an funktionelle Bissregistrate, wobei auch eine Anpassung der Zahnmodelle an gewünschte Kontaktpunkte, -flächen möglich ist. Bei der Berechnung dieser Optimierungsteilwerte kann auch die Kieferbewegung mit einbezogen werden. Ein weiterer bevorzugter Optimierungsteilwert beschreibt die Anpassung der Zahnmodelle an die Nachbarzähne, wobei neben der Anpassung an gewünschte Kontaktpunkte, -flächen vorzugsweise auch ein optimaler Interdentalraum gebildet werden soll. Angestrebt ist eine Form, bei der sich möglichst keine Essensreste festsetzen und gleichzeitig eine gute Zahnreinigung möglich ist. Sofern Präparations- und/oder Segmentierungslinien und/oder anatomische Landmarken in den Scandaten vorhanden sind, können auch Optimierungsteilwerte für die Zahnmodelle berechnet werden, welche die Anpassung der Zahnmodelle an die Präparations- und/oder Segmentierungslinien und/oder anatomische Landmarken beschreiben. Ein weiteres Optimierungskriterium betrifft die mechanische Stabilität der Zahnrestaurationen, die sich aus den Zahnmodellen und Scandaten ergeben. Der dazugehörige Optimierungsteilwert beschreibt vorzugsweise die Einhaltung minimaler Materialstärken, um die mechanische Stabilität der Zahnrestaurationen zu gewährleisten. Neben diesen beispielhaft aufgeführten funktionalen Optimierungskriterien können auch ästhetische Optimierungskriterien berücksichtigt werden, welche vor allem für Zahnrestaurationen im Frontzahnbereich wichtig sind. So kann der Patient eine besondere Form (rechteckig, dreieckig, quadratisch, schaufelförmig, etc.) der oberen Schneidezähne bevorzugen. Der dazugehörige Optimierungsteilwert beschreibt die Abweichungen der Zahnmodelle zu den bevorzugten Formen.

Die zur Individualisierung von Zahnmodellen benötigte Zielstrukturen (Zähne, Restzahnsubstanzen, Gegenbezahnung, Bissregistrate, Nachbarzähne, Präparations-, Segmentierungslinien, anatomische Landmarken etc.) können algorithmisch und/oder interaktiv in den Scandaten bestimmt werden. Entsprechende Verfahren sind dem Fachmann bekannt, wobei insbesondere automatisierte Verfahren von Vorteil sind, da sie ein objektives und zeitsparendes Vorgehen ermöglichen.

Wie oben beschrieben können auch Gruppen von Zahnmodellen individualisiert werden, wobei dann vorzugsweise Zahnmodelle innerhalb einer Gruppe gekoppelt werden. Zu jeder so gebildeten Kopplungsgruppe kann ein weiterer Optimierungsteilwert gehören.

Der Umfang einer Kopplungsgruppe kann grundsätzlich durch eine beliebige Menge unterschiedlicher Zahntypen aus der Menge der gewünschten Zahntypen festgelegt (z.B. durch die Zahntypen 13 bis 18 oder z.B. durch die Zahntypen 15 und 26) werden, d.h. die dazugehörigen Zahnmodelle müssen nicht notwendigerweise benachbart sein. Sind Zahnmodelle benachbart, so werden die benachbarten Zahnmodelle vorzugsweise derart gekoppelt, dass der dazugehörige Optimierungsteilwert die Kontaktsituation der benachbarten Zahnmodelle beschreibt. Darüber hinaus können innerhalb einer Kopplungsgruppe die Lagen und/oder Formen der einzelnen Zahnmodelle derart gekoppelt werden, dass anatomisch sinnvolle Beziehungen der Zahnmodelle untereinander eingehalten werden. Dazu können Kopplungsparameter der Kopplungsgruppe Relationsbeziehungen für die Lage- und/oder Formparameter der einzelnen Zahnmodelle definieren. Der Optimierungsteilwert für die relativen Lagen von Zahnmodellen ergibt sich vorzugsweise aus den räumlichen Relationen von anatomischen Landmarken der Zahnmodelle. Die Kopplungsparameter können dabei gewünschte Relationen der Landmarken untereinander beschreiben und aus den Abweichungen von diesen Relationen kann der Optimierungsteilwert für die Lagebeziehungen berechnet werden. Als Beispiel sei hier die Ausrichtung von Zahnmodell-Schneidekanten angeführt. Obwohl die Formen der Zahnmodelle hier stark variieren können (rechteckig, dreieckig, etc.), ergibt die Forderung, dass die Landmarken der Zahnmodell-Schneidekanten auf einer parametrisierten Kurve liegen sollen, eine gute Ausrichtung der Zahnmodell-Schneidekanten im Frontzahnbereich. Ähnlich verhält es sich mit einem Optimierungsteilwert, der die räumlichen Relationen der Formen von Zahnmodellen beschreibt. Vorzugsweise kann eine Modelldatenbank aus Kieferscans von Probanden aufgebaut werden, wobei die aus einem Kieferscan erzeugten Zahnmodelle untereinander korrespondieren, da sie alle von einem Probanden stammen. Der Optimierungsteilwert für die Kopplung der Formen der Zahnmodelle kann die Abweichungen von den Korrespondenzen innerhalb einer Kopplungsgruppe beschreiben.

Der Umfang zumindest einer Kopplungsgruppe sollte vorzugsweise dem Umfang der gescannten Zähne entsprechen, damit möglichst viel Zahn- und Restzahnsubstanz durch das Verfahren analysiert wird. Insbesondere bei vorhandenen Zahnpräparationen wird aus der Zahn- und Restzahnsubstanz aller gescannten Zähne auf die Formen der Zahnrestaurationen geschlossen. Je größer dabei der Zahndefekt für einen Zahntyp ist, desto wichtiger ist die Analyse der Zahn- und Restzahnsubstanzen anderer Zahntypen. Insbesondere wenn aber nur ein minimaler Defekt vorliegt, z.B. bei einer Inlaypräparation ohne Höckerbeteiligung, so funktioniert das erfindungsgemäß vorgeschlagene Verfahren auch mit einem einzigen Zahntyp ausgezeichnet. Alternativ können aber auch bei der Verwendung von mehreren Zahntypen die Kopplungs-Optimierungsteilwerte von Zahntypen mit geringen Zahndefekten sehr gering oder sogar mit Null gewichtet sein.

Die einzelnen Optimierungsteilwerte werden schließlich zu einem einzigen Optimierungswert für die Individualisierung zusammengefasst. Vorzugsweise geschieht dies durch die Berechnung einer gewichteten Summe der Optimierungsteilwerte. Die Gewichte ermöglichen dabei eine Steuerung des Einflusses der einzelnen Optimierungskriterien. So erhält man erfindungsgemäß für einen Modellparameter-Satz einen Optimierungswert. Zur Individualisierung eines Zahnmodells werden dann Modellparameter solange variiert, bis der Optimierungswert minimal oder ein Abbruchkriterium erfüllt ist. Der Qualitätswert der Individualisierung wird vorzugsweise aus dem minimalen Optimierungswert bestimmt.

Bei einer bevorzugten Variante des Verfahrens wird nach der Bestimmung der optimalen Zahnmodelle und vor Bestimmung der Zahnrestaurationen eine Feinanpassung der optimalen Zahnmodelle untereinander und/oder an die Scandaten durchgeführt. Dies ist vorteilhaft, da die Formenvielfalt der Zahnmodelle durch die Anzahl der Zahnmodelle in der Modelldatenbank und die Anzahl der Formparameter praktisch beschränkt ist und die berechneten Zahnrestaurationen eine maximale Präzision aufweisen sollen. Von Vorteil ist die Anwendung lokal beschränkter Deformationstransformationen, welche eine Feinanpassung der Kontaktsituation der Zahnmodelle untereinander sowie eine Feinanpassung an die Zähne, Restzahnsubstanzen, Nachbarzähne, Gegenbezahnung, Bissregistrate, Präparationslinien, Segmentierungslinien, anatomischen Landmarken, Kontaktpunkte etc. mit möglichst geringen Verschiebungswerten vornehmen und dabei keine Kanten oder Faltungen erzeugen.

Erfindungsgemäß kann die Suche nach dem optimalen Zahnmodell eines Zahntyps bei einem oder bei mehreren Start-Zahnmodellen beginnen. Bei mehreren Start-Zahnmodellen erhält man für jedes einzelne Start-Zahnmodell durch das Verfahren ein temporäres optimales Zahnmodell mit einem Qualitätswert für die Individualisierung. Das endgültige optimale Zahnmodell ist dann dasjenige temporäre optimale Zahnmodell mit dem besten Qualitätswert.

Insbesondere bei der Verwendung eines einzigen Start-Zahnmodells kann das Start-Zahnmodell in einer Modelldatenbank festgelegt sein, wobei dieses Start-Zahnmodell vorzugsweise das Mittelwert-Zahnmodell des Zahntyps sein sollte.

Ebenso kann das Start-Zahnmodell auch aus einer geometrischen Analyse der Scandaten bestimmt werden. Dazu können geometrische Maße der in den Scandaten vorhandenen Zähne und/oder der Restzahnsubstanzen ermittelt und daraus ein Start-Zahnmodell mit möglichst ähnlichen geometrischen Maßen aus einer Modelldatenbank bestimmt werden.

Die Auswahl mehrerer Start-Zahnmodelle für einen Zahntyp kann analog zur Auswahl eines einzigen Start-Zahnmodells durchgeführt werden. Dabei ist es am einfachsten, die Start-Zahnmodelle in einer vom Verfahren verwendeten Modelldatenbank festzulegen, wobei es auch hier vorteilhaft ist, wenn die Start-Zahnmodelle Mittelwert-Zahnmodelle von Untergruppen der Zahnmodelle eines Zahntyps sind. Dabei sollten die Untergruppen aus morphologisch ähnlichen Zahnmodellen des Zahntyps bestehen. Sollen die Start-Zahnmodelle durch eine geometrische Analyse der Scandaten bestimmt werden, so wird eine Anzahl von Start-Zahnmodellen aus der Modelldatenbank ausgewählt, deren geometrische Maße möglichst gut zu den geometrischen Maßen von Zähnen und/oder von Restzahnsubstanzen in den Scandaten korrespondieren.

Eine weitere bevorzugte Variante des erfindungsgemäßen Verfahrens ist dadurch gekennzeichnet, dass die vorhandenen Zahntypen einer Modelldatenbank zur Auswahl einer Auswahleinheit zugeführt werden und mit Hilfe eines Auswahlsignals, beispielsweise durch die Verwendung einer grafischen Benutzeroberfläche, die gewünschten Zahntypen selektiert werden. Diese Selektion kann durch die Verwendung eines grafischen Zahnschemas übersichtlich gestaltet werden.

Damit auch beeinflusst werden kann, welche Zahnmodelle einer Modelldatenbank bei dem erfindungsgemäßen Verfahren verwendet werden, werden vorzugsweise zusätzlich oder alternativ die in der Modelldatenbank vorhandene Zahnmodelle eines gewünschten Zahntyps zur Auswahl einer Auswahleinheit zugeführt und mit Hilfe eines Auswahlsignals gewünschte Zahnmodelle selektiert und aus diesen die optimalen Zahnmodelle bestimmt. Z.B. können besonders bevorzugt die Struktur der Verlinkung von Zahnmodellen einer Modelldatenbank und/oder eine lineare Sortierung von Zahnmodellen einer Modelldatenbank zur Auswahl einer Auswahleinheit zugeführt werden und mit Hilfe eines Auswahlsignals gewünschte Zahnmodelle selektiert werden. Von Vorteil ist dieses Vorgehen insbesondere bei der Auswahl von Zahnmodellen gewünschter Formen im Frontzahnbereich oder bei der Auswahl von Zahnmodellen für die Prothesenherstellung.

Die Zahnmodelle einer Modelldatenbank liegen bei einer bevorzugten Variante des erfindungsgemäßen Verfahrens in verschiedenen Auflösungsstufen vor. Es ist dann von Vorteil, wenn das erfindungsgemäße Verfahren mit den Zahnmodellen der gröbsten Auflösungsstufe beginnt, wobei die Auflösungsstufe in späteren Schritten, nach erfolgreichem Durchlauf, erhöht wird. Dies ermöglicht vor allem eine Geschwindigkeitssteigerung des Verfahrens. Dabei kann die Datenbank z.B. auch komplette Sätze von Zahnmodellen in unterschiedlichen Auflösungsstufen umfassen, bzw. Teildatenbanken mit jeweils unterschiedlichen Auflösungsstufen, und es wird nach einer kompletter Optimierung, d.h. nach Auswahl eines optimalen Zahnmodels aus einer Daten- oder Teildatenbank mit einer niedrigen Auflösungsstufe das Verfahren noch einmal mit einer Daten- oder Teildatenbank mit einer höheren Auflösungsstufe durchgeführt. Dabei kann das optimale Zahnmodell aus einem vorhergehenden Durchlauf als Startmodell für den nachfolgenden Durchlauf verwendet werden. Ebenso kann bei der Lösung der Optimierungsaufgabe pro Iterationsschritt jeweils mit verschiedenen Auflösungsstufen gearbeitet werden.

Die geometrisch deformierbaren Zahnmodelle in einer Modelldatenbank können nach verschiedenen Prinzipien aufgebaut sein. Da es sich bei den Zielstrukturen in den Scandaten vor allem um Grenzflächen handelt, bieten sich Oberflächenmodelle als Zahnmodelle an. Die geometrische Modellierung der Modelloberflächen kann durch einfache Triangulierung erfolgen, oder aber auch durch aufwändigere Modellierungen höherer Ordnung (Bezier-, Nurbs-, B-Spline-Modelle, etc.). Mit mehr Rechen- und Speicheraufwand als bei den Oberflächenmodellen ist hingegen die Verwendung von Volumenmodellen (Voxel-, FEM-Modellen, etc.) verbunden. Mit ihnen lassen sich gut innere Strukturen von Zähnen aber auch mechanische Eigenschaften modellieren. Von Vorteil ist die Markierung von anatomischen Landmarken, Zahnachsen, Richtungsbezeichnungen, Oberflächenregionen und/oder weiteren charakteristischen geometrischen Strukturen an den Zahnmodellen, da man diese Markierungen nach erfolgreicher Individualisierung der Zahnmodelle auf die Scandaten übertragen kann.

Die geometrischen Transformationen von Zahnmodellen werden durch Formparameter beschrieben, wobei diese Parametrisierung auf unterschiedliche Weise realisiert werden kann. Vorzugsweise finden Transformationen Verwendung, bei denen die Formparameter der Zahnmodelle entsprechend ihres Einflusses auf die Zahnmodellgeometrie geordnet sind. D.h. die wichtigen Formparameter stehen am Anfang der Formparameterliste und werden bei der Zahnmodellindividualisierung zuerst optimiert, bevor Formparameter optimiert werden, die Details der Zahnmodelle parametrisieren. Dadurch wird eine Geschwindigkeitssteigerung und Stabilisierung der Zahnmodellindividualisierung erreicht. Der Definitionsbereich der Formparameter ist dabei vorzugsweise nicht beliebig, sondern wird bevorzugt durch die Analyse von Trainingsdaten bestimmt. Auf diese Weise wird erreicht, dass nur anatomisch sinnvolle Zahnmodelle durch die Formparameter erzeugt werden.

Von besonderem Vorteil für das erfindungsgemäß vorgeschlagene Verfahren ist die Verwendung von parametrisierten dreidimensionalen Transformationsfeldern für die Zahnmodelle. Ein dreidimensionales Transformationsfeld besteht aus Verschiebungsvektoren für die Vertices (Stützpunkte) eines Zahnmodells. Diese Verschiebungsvektoren können auch parametrisiert werden, wie z.B. durch die parametrisierte Verschiebung von anatomischen Landmarken eines Zahnmodells. Die Verschiebungsvektoren aller Vertices ergeben sich dann beispielsweise aus den Entfernungen der Vertices zu den verschobenen anatomischen Landmarken. Hiermit lässt sich neben dem Morphing von Zahnmodellen eine Reduktion des Speicherumfangs der Modelldatenbank erreichen, da diejenigen Zahnmodelle aus der Modelldatenbank entfernt werden können, die sich aus einem anderen Zahnmodell unter Anwendung einer parametrisierten dreidimensionalen Transformation in hinreichender Genauigkeit ergeben. Hingegen ist bei Anwendungen des vorgeschlagenen Verfahrens, bei denen weniger die Ansprüche an die Genauigkeit, sondern eher schnell zu erzielende Ergebnisse im Vordergrund stehen, erfindungsgemäß die Verwendung von Formparametern vorteilhaft, die geometrischen Konstruktionsparametern entsprechen (z.B. Zahnbreite, -tiefe, Höckerabstände, Schneidekantendicke, Wurzellängen, etc.).

Das erfindungsgemäß vorgeschlagene Verfahren kann aber, wie eingangs erläutert, insbesondere auch mit geometrisch nicht deformierbaren Zahnmodellen verwendet werden. Dies ist dann sinnvoll, wenn die Zahnmodelle vorkonfektionierten künstlichen Zähnen, Zahngruppen oder Gebissen entsprechen, die vor Anwendung des Verfahrens massenhaft und kostengünstig produziert werden. Als Beispiel sei hier die Prothesenherstellung auf Basis einer Bibliothek vorgegebener Prothesenzähne genannt.

Bei einem erfindungsgemäßen Verfahren zur Erzeugung einer Modelldatenbank, die zur Verwendung in dem oben beschrieben Verfahren für verschiedene Zahntypen jeweils eine Anzahl von parametrisierten Zahnmodellen enthält, erfolgt die Parametrisierung anhand von Modellparametern, die Lage- und/oder Formparameter umfassen. Außerdem wird jedes Zahnmodell mit einer Anzahl von Zahnmodellen desselben Zahntyps verlinkt. Eine solche Modelldatenbank für das erfindungsgemäße Verfahren kann dabei auf verschiedene Arten erzeugt werden. Von Vorteil sind automatisierte Verfahren zum Aufbau einer Modelldatenbank, da insbesondere bei einer größeren Anzahl von Zahnmodellen eines Zahntyps die interaktive Erstellung einer Verlinkung und der geometrischen Transformationen der Zahnmodelle subjektiv und sehr zeitaufwändig sein kann.

Nach einem bevorzugten Verfahren werden dabei die ganze Modelldatenbank oder zumindest Teile der Modelldatenbank durch die Analyse einer Menge von Scandaten künstlicher und/oder natürlicher oraler Strukturen aufgebaut. Künstliche orale Strukturen umfassen im Sinne der Erfindung künstliche Zähne, Zahngruppen, Gebisse aber auch Implantate, Abutments und weitere Verankerungssysteme. In der Regel liegen solche künstlichen oralen Strukturen in einer Vielzahl vorkonfektionierter Ausprägungen von verschiedenen kommerziellen Anbietern vor. Hingegen umfassen natürliche orale Strukturen im Sinne der Erfindung natürliche Zähne, Zahngruppen, Gebisse aber auch Zahnfleisch, Kieferknochen, Nerven und weitere anatomische Strukturen. Vorzugsweise erhält man die Scandaten natürlicher oraler Strukturen aus optischen Scans von Gipsabdrücken karies-, bzw. defektfreier Ober- und Unterkiefer. Es können aber auch radiologische Scandaten von Kiefern verwendet werden, wobei die Scandaten eine geringere Genauigkeit als die optischen Scandaten aufweisen, dafür aber subgingivale und intradentale Strukturen enthalten.

In der Regel müssen die Scandaten, insbesondere wenn es sich um Scandaten natürlicher oraler Strukturen handelt, in einem ersten Verfahrensschritt zuerst segmentiert werden, um Scandaten eines gewünschten Zahntyps zu erhalten. Dieser Schritt entfällt meist bei Scandaten künstlicher oraler Strukturen, da diese in der Regel physisch separiert vorliegen und einzeln eingescannt werden können. In beiden Fällen erhält man aus der Vermessung physisch vorhandener oraler Strukturen eine Menge von Scandaten eines gewünschten Zahntyps, die zum Aufbau einer Modelldatenbank verwendet werden kann.

In einem anschließenden Verfahrensschritt werden erfindungsgemäß die Zahnmodelle einer Modellklasse konstruiert, d.h. es findet eine Anpassung von Oberflächen- bzw. Volumen-Zahnmodellen (einer gewünschten Auflösung) an die Scandaten eines gewünschten Zahntyps statt. Optional können anatomische Landmarken, Zahnachsen, Richtungsbezeichnungen, Oberflächenregionen und/oder weitere charakteristische geometrische Strukturen für die Zahnmodelle bestimmt werden.

Eine erfindungsgemäße Verlinkung der Zahnmodelle einer Modellklasse kann besonders bevorzugt dadurch erzeugt werden, dass Cluster von morphologisch ähnlichen Zahnmodellen gebildet werden, wobei die Zahnmodelle eines Clusters sowie die Cluster untereinander verlinkt sind.

Zur Bestimmung von Clustern mit den dazugehörigen Mittelwert-Zahnmodellen kann zuvor eine Analyse von morphologischen Abweichungen der Zahnmodelle untereinander durchgeführt werden, indem für jedes mögliche Paar von Zahnmodellen ein Abweichungswert berechnet wird. Dies kann dadurch erreicht werden, dass die beiden Zahnmodelle eines Paars durch die Bestimmung optimaler Translations- und Rotationswerte möglichst gut zur Deckung gebracht werden und dass der Abweichungswert die noch bestehende morphologische Abweichung der Zahnmodelle beschreibt.

Zahnmodelle werden vorzugsweise als morphologisch ähnlich betrachtet und dann untereinander verlinkt, wenn die dazugehörigen Abweichungswerte unterhalb eines Schwellenwerts liegen. D.h. je nach vorgegebenem Schwellenwert zerfällt die Menge aller Zahnmodelle in Cluster. Je kleiner dabei die Schwelle gewählt wird, desto größer ist die Anzahl der Cluster, wobei die Cluster in diesem Stadium definitionsgemäß noch nicht miteinander verlinkt sind.

Eine Verlinkung der Cluster kann vorzugsweise auf der Basis von Mittelwert-Zahnmodellen der Cluster stattfinden. Aufgrund der Abweichungsanalyse der Zahnmodelle untereinander erhält man für jedes der n Zahnmodelle eines Clusters (n-1) Abweichungswerte. Als Mittelwert-Zahnmodell wird vorzugsweise dasjenige Zahnmodell angesehen, dessen Summe der Quadrate der Abweichungswerte minimal ist. Nach der Bestimmung der Mittelwert-Zahnmodelle der Cluster werden diese geeignet verlinkt, damit eine zusammenhängende Verlinkung aller Zahnmodelle der Modellklasse aufgebaut wird. Dabei wird vorzugsweise das Mittelwert-Zahnmodell des umfangstärksten Clusters mit allen anderen Mittelwert-Zahnmodellen der anderen Cluster verlinkt. Zusätzlich kann auch noch eine Verlinkung von Mittelwert-Zahnmodellen der Cluster hinzugefügt werden, die morphologisch ähnlich sind. Bei einer größeren Anzahl von Zahnmodellen bietet sich eine iterative Anwendung dieses Verfahrens an. Cluster, die einen gewissen Umfang überschreiten, werden durch das beschriebene Verfahren in Cluster 2. Ordnung unterteilt und die dazugehörigen Zahnmodelle entsprechend verlinkt.

Insbesondere bei Verwendung von Scandaten natürlicher oraler Strukturen zum Aufbau zumindest eines Teils der Modelldatenbank werden in einem abschließenden Verfahrensschritt vorzugsweise geometrische Transformationen zu den Zahnmodellen hinzugefügt, um auch Zwischenformen von Zahnmodellen für die Individualisierung zu erzeugen. Für ein Zahnmodell werden geeignete geometrische Transformationen bevorzugt durch die Forderung definiert, dass die Form des Zahnmodells in zumindest ein mit ihm verlinktes Zahnmodell kontinuierlich überführt werden kann. Vorzugsweise sind dabei die Formparameter gemäß ihres Einflusses auf die Zahnmodellgeometrie geordnet. Durch dieses Vorgehen ist sichergestellt, dass ein Morphing nur zwischen morphologisch ähnlichen Zahnmodellen stattfindet und dass die dazugehörigen Formparameter effektiv variiert werden können.

Ein erfindungsgemäßes Zahnrestaurationsermittlungssystem benötigt zur Umsetzung des Verfahrens eine Schnittstelle zum Empfang der von einer Modalität gemessenen Scandaten, eine Auswahleinheit zur Auswahl der vom Verfahren zu verwendenden Zahntypen, eine Speichereinrichtung mit einer Modelldatenbank, die für verschiedene Zahntypen jeweils eine Anzahl von parametrisierten Zahnmodellen enthält, wobei die Parametrisierung anhand von Modellparametern erfolgt, welche Lage- und/oder Formparameter umfassen und wobei jedes Zahnmodell mit einer Anzahl von Zahnmodellen desselben Zahntyps verlinkt ist. Außerdem benötigt das Zahnrestaurationsermittlungssystem eine Optimierungseinheit, welche ausgebildet ist, um für jeden gewünschten Zahntyp ein optimales Zahnmodell aus der Modelldatenbank durch ein iteratives Verfahren zu bestimmen, bei dem beginnend mit einem Start-Zahnmodell bei jedem Iterationsschritt ein Zahnmodell hinsichtlich eines Qualitätswerts getestet wird. Die Optimierungseinheit umfasst dabei eine Ladeeinheit, eine Individualisierungseinheit und eine Qualitätsermittlungseinheit. Mittels der Ladeeinheit werden Zahnmodelle aus einer Modelldatenbank geladen und der Individualisierungseinheit zugeführt, welche ausgebildet ist, um ein aktuell zu testendes Zahnmodell und zumindest ein mit dem zu testenden Zahnmodell verlinktes Zahnmodell durch Variation von Modellparametern an die Scandaten anzupassen. Innerhalb der Qualitätsermittlungseinheit werden Qualitätswerte für die Individualisierung der Zahnmodelle ermittelt und ein Qualitätskriterium für den Abbruch der Iteration überprüft. Schließlich benötigt das Zahnrestaurationsermittlungssystem eine Restaurationseinheit, um aus den optimalen Zahnmodellen und Scandaten zumindest eine Zahnrestauration zu bestimmen.

Auswahl-, Optimierungs-, Lade-, Individualisierungs-, Qualitätsermittlungs- und Restaurationseinheit des Zahnrestaurationsermittlungssystems können besonders bevorzugt in Form von Software auf einem entsprechend geeigneten Prozessor eines Computers realisiert werden. Dieser Computer sollte eine entsprechende Schnittstelle zum Empfang der Scandaten und eine geeignete Speichereinrichtung für eine Modelldatenbank aufweisen. Dabei muss diese Speichereinrichtung nicht notwendigerweise integrierter Bestandteil des Computers sein, sondern es reicht aus, wenn der Computer auf eine passende externe Speichereinrichtung zugreifen kann. Eine Realisierung des erfindungsgemäßen Verfahrens in Form von Software hat den Vorteil, dass auch bestehende Zahnrestaurationsermittlungssysteme relativ einfach durch geeignete Updates entsprechend nachgerüstet werden können. Bei dem erfindungsgemäßen Zahnrestaurationsermittlungssystem kann es sich insbesondere auch um eine Ansteuerungseinheit für die Scandaten selbst aufzeichnende Modalität handeln, welche die notwendigen Komponenten zur erfindungsgemäßen Bearbeitung der Scandaten aufweist.

Ebenso kann auch ein separates, der Ermittlung von Zahnrestaurationen vorgeschaltetes erfindungsgemäßes Verfahren zur Erzeugung zumindest von Teilen einer Modelldatenbank in Form von geeigneter Software auf einem Computer realisiert werden. Insbesondere ist es dabei auch möglich, das Zahnrestaurationsermittlungssystem, mit welchem die Ermittlung von Zahnrestaurationen durchgeführt wird, zur Erzeugung von zumindest Teilen einer Modelldatenbank zu nutzen. Beispielsweise könnten zu bestimmten Zeiten, in denen das Zahnrestaurationsermittlungssystem nur wenig durch aktuelle Arbeiten ausgelastet ist, freie Rechenkapazitäten verwendet werden, um Zahnmodelle zu erzeugen und in der Modelldatenbank für eine spätere Nutzung zu hinterlegen.

Das erfindungsgemäße Verfahren und das erfindungemäße Zahnrestaurationsermittlungssystem zur Ermittlung von Zahnrestaurationen können vorteilhafterweise in einem erfindungsgemäßen Verfahren zur Herstellung oder Auswahl eines Zahnrestaurationsteils eingesetzt werden. Hierbei wird zunächst auf der Basis der Scandaten oraler Strukturen eine virtuelle Zahnrestauration wie oben beschrieben ermittelt. Anschließend kann basierend auf der ermittelten virtuellen Zahnrestauration das Zahnrestaurationsteil hergestellt werden. Dies erfolgt vorzugsweise automatisch mittels einer Maschine, beispielsweise einer Fräseinrichtung, an die beispielsweise ein CAD/CAM-Datensatz übermittelt wird, der die virtuelle Zahnrestauration beschreibt. Alternativ kann auch ein passendes Zahnrestaurationsteil aus einer Menge von vorgefertigten Zahnrestaurationsteilen ausgewählt werden, z.B. durch Ausgabe einer Identifikationsnummer, welche dem Zahnrestaurationsteil eindeutig zugeordnet ist.

Die Erfindung wird im Folgenden anhand von Ausführungsbeispielen unter Hinweis auf die beigefügten Zeichnungen näher erläutert. Es zeigen:
Fig. 1 ein Flussdiagramm zur Darstellung eines möglichen Ablaufs des erfindungsgemäßen Verfahrens zur Ermittlung von Zahnrestaurationen,
Fig. 2 eine schematische Darstellung einer Modelldatenbank DB mit verlinkten Zahnmodellen M für zwei Zahntypen 15, 16,
Fig. 3 eine perspektivische Darstellung von Mittelwert-Zahnmodellen M einer Modelldatenbank für Zahntypen 18 bis 48,
Fig. 4 eine zweidimensionale schematische Darstellung von Scandaten D von Zähnen **T₁₃** bis **T₁₈** mit Antagonisten-Daten, markierten Präparationslinien LP, markierten Segmentierungslinien LS und markierten anatomischen Landmarken AL,
Fig. 5 eine zweidimensionale schematische Darstellung von Scandaten D von Zähnen **T₁₃** bis **T₁₈** mit optimalen Zahnmodellen M für eine Inlay-, Kronen- und Brückenpräparation,
Fig. 6 eine zweidimensionale schematische Darstellung von Scandaten D von Zähnen **T₁₃** bis **T₁₈** mit Zahnrestaurationen R für eine Inlay-, Kronen- und Brückenpräparation,
Fig. 7 eine perspektivische Darstellung von Scandaten D eines Kiefers, die zum Aufbau einer Modelldatenbank verwendet werden, wobei in der linken Bildhälfte der Oberkieferscan, und rechts der Unterkieferscan abgebildet ist,
Fig. 8 eine perspektivische Darstellung von Zahnmodellen M einer Modellklasse, die durch Anpassung an aus der Segmentierung von Kieferscans stammenden Scandaten eines Zahntyps konstruiert wurden,
Fig. 9 eine perspektivische Darstellung einer Aufstellung von Zahnmodellen M einer Modellklasse, definiert durch die gegenseitigen Abweichungswerte, wobei morphologisch ähnliche Zahnmodelle M eine geringe räumliche Distanz bei der Aufstellung zueinander aufweisen,
**Fig. 10** eine perspektivische Darstellung einer möglichen Verlinkung **L** von Zahnmodellen **M** einer Modellklasse mit einem einzigen Cluster **C₁**,
**Fig. 11** eine perspektivische Darstellung einer möglichen Verlinkung **L** von Zahnmodellen **M** einer Modelldatenbank mit vier Clustern **C₁, C₂, C₃, C₄,**
**Fig. 12** eine perspektivische Darstellung einer möglichen linearen Sortierung **P** von Zahnmodellen **M** einer Modellklasse durch Verbindung von Zahnmodellen **M** mit den geringsten morphologischen Unterschieden,
**Fig. 13** eine perspektivische Darstellung einer eindimensionalen Aufstellung von Zahnmodellen **M** einer Modellklasse, definiert durch eine lineare Sortierung und Clusterzugehörigkeit **C₁, C₂, C₃, C₄** gemäß **Fig. 11****,**
**Fig. 14** eine perspektivische Darstellung einer zweidimensionalen Aufstellung von Zahnmodellen **M einer** Modellklasse, definiert durch eine lineare Sortierung und Clusterzugehörigkeit **C₁, C₂, C₃, C₄** gemäß **Fig. 11****,**
**Fig. 15** eine Darstellung eines Zahnschemas, mit dem die vom Verfahren zu verwendenden Zahntypen und optional auch die dazugehörigen Präparationstypen definiert werden,
**Fig. 16** eine reduzierte Darstellung des Zahnschemas von **Fig. 15****,**
**Fig. 17** ein schematisch dargestelltes Ausführungsbeispiel eines erfindungsgemäßen Zahnrestaurationsermittlungssystems.

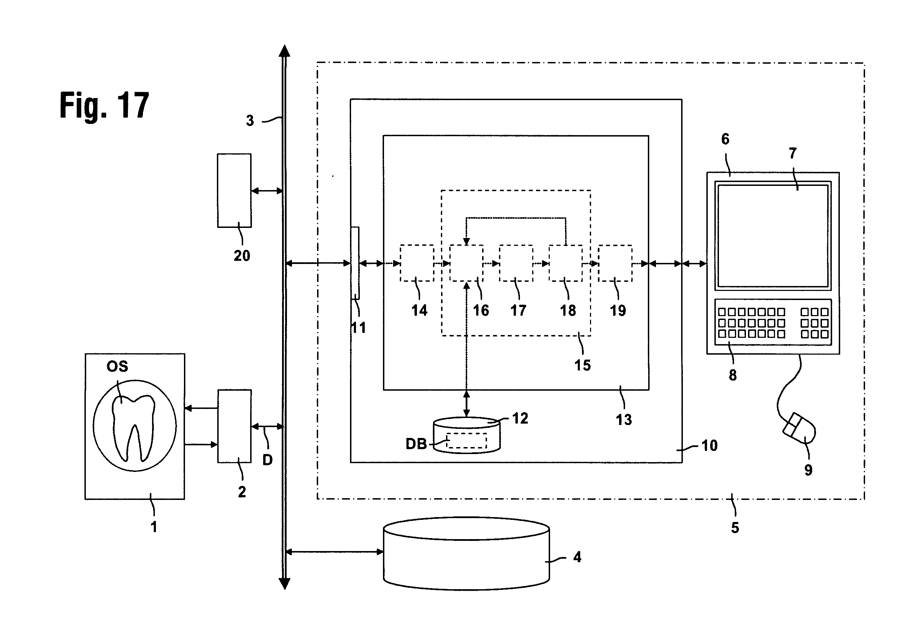

Das in **Fig. 17** schematisch dargestellte Ausführungsbeispiel eines erfindungsgemäßen Zahnrestaurationsermittlungssystems **5** besteht im Wesentlichen aus einem Computer **10** und einer daran angeschlossenen Konsole **6** o.Ä. mit einem Bildschirm **7,** einer Tastatur **8** und einer Zeigeeinrichtung, hier einer Maus **9.** Bei dem Computer **10** kann es sich um einen in üblicher Weise aufgebauten Computer, beispielsweise einem PC oder einer Workstation handeln, welcher auch zu sonstigen Datenauswertungen und/oder zur Steuerung von Bildaufnahmegeräten (Modalitäten) wie optischen Scannern, Digitalen Volumentomographen, Computertomographen, etc. eingesetzt werden kann. Wesentliche Komponenten innerhalb dieses Computers **10** sind u.a. ein zentraler Prozessor **13** und eine Schnittstelle **11,** um Scandaten **D** oraler Strukturen **OS** zu empfangen, welche von einer Modalität **1,** hier einem optischen Scanner, gemessen wurden. Über die Schnittstelle **11** können aber auch vom Verfahren ermittelte CAD/CAM-Datensätze an eine an den Datenbus **3** angeschlossene Schleifeinheit **20** ausgegeben werden, mit der z.B. Zahnrestaurationsteile erzeugt werden können.

In dem in **Fig. 17** dargestellten Ausführungsbeispiel ist die Modalität **1** mit einer Steuereinrichtung **2** verbunden, welche wiederum mit einem Datenbus **3** verbunden ist, an dem auch das Zahnrestaurationsermittlungssystem **5** angeschlossen ist. Außerdem sind an diesem Datenbus **3** ein Massenspeicher **4** zur Zwischenspeicherung oder dauerhaften Hinterlegung der von der Modalität **1** aufgezeichneten Scandaten **D** und/oder der von dem Zahnrestaurationsermittlungssystem **5** weiter verarbeiteten Scandaten **D** angeschlossen. Selbstverständlich können am Datenbus **3** unter Bildung eines größeren Netzwerks noch andere Komponenten, beispielsweise weitere Modalitäten, Massenspeicher, Workstations, Ausgabegeräte wie Drucker, Filming-Stationen, Schleifeinheiten **20** o.Ä. angeschlossen sein. Ebenso ist eine Verbindung mit einem externen Netz bzw. mit weiteren Zahnrestaurationsermittlungssystemen möglich. Sämtliche Scandaten **D** werden dabei vorzugsweise zur Kommunikation im sog. STL-Standard (STL = Surface Tesselation Language) und/oder im DICOM-Standard (DICOM = Digital Imaging and Communication in Medicine) formatiert.

Die Ansteuerung der Modalität **1** erfolgt in üblicher Weise über die Steuereinrichtung **2,** welche auch die Daten von der Modalität **1** akquiriert. Die Steuereinrichtung **2** kann zur Bedienung vor Ort eine eigene Konsole oder Ähnliches aufweisen, die hier jedoch nicht dargestellt ist. Es ist aber auch möglich, dass die Bedienung beispielsweise über den Datenbus mittels einer separaten Workstation erfolgt, welche sich in der Nähe der Modalität befindet.

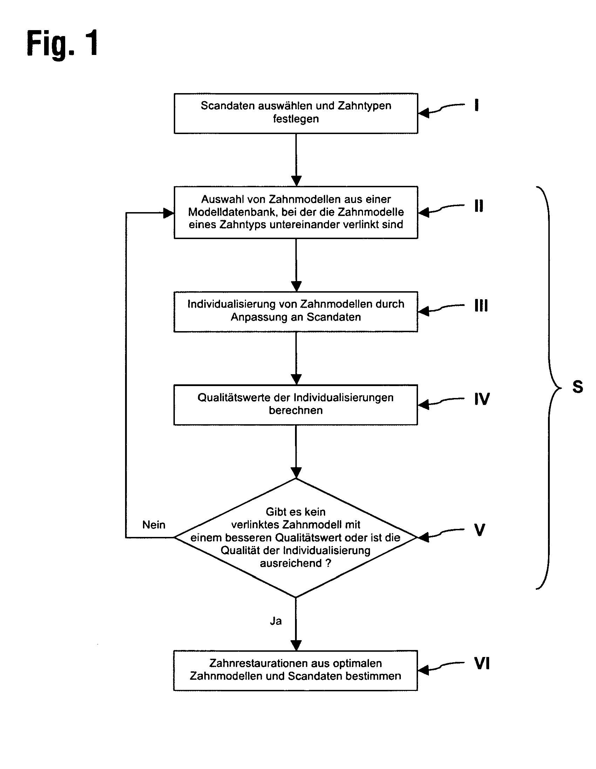

Ein möglicher Ablauf des erfindungsgemäßen Verfahrens wird im Folgenden anhand von in **Fig. 1** erläutert, wobei bezüglich des Aufbaus des Zahnrestaurationsermittlungssystems auf **Fig. 17** verwiesen wird. Das Verfahren besteht hier aus den Verfahrensschritten **I** bis **VI**, wobei ein Iterationsschritt **S** aus den Verfahrensschritten **II** bis **V** besteht. Dabei wird im Folgenden davon ausgegangen, dass eine gemeinsame Ermittlung optimaler Zahnmodelle für eine Gruppe von Zahntypen erfolgt, auch wenn gemäß der Erfindung prinzipiell auch für jeden Zahntyp separat ein optimales Zahnmodel ermittelt werden könnte.

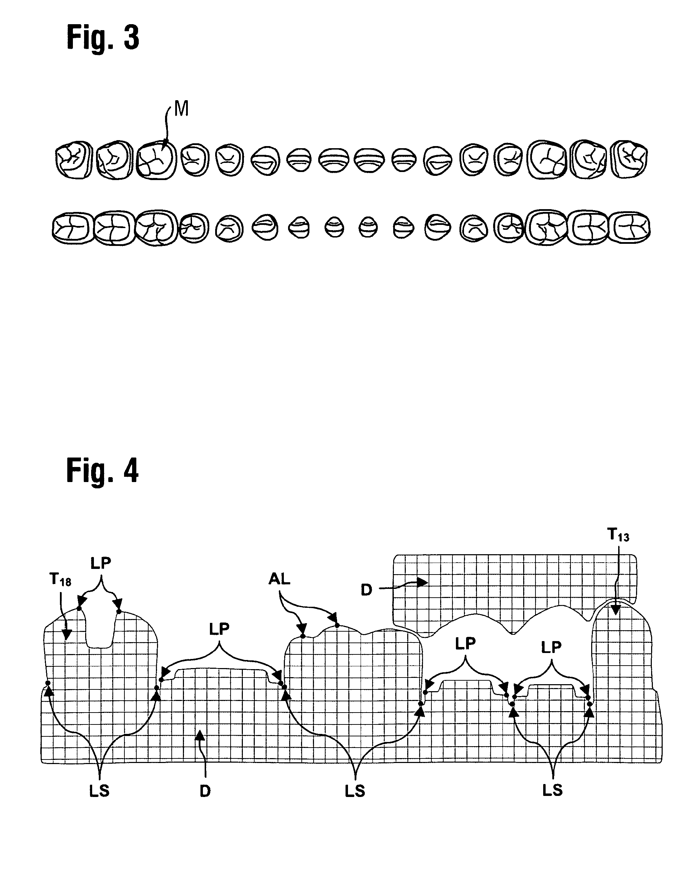

Zunächst werden in einem ersten Verfahrensschritt **I** die Scandaten **D** ausgewählt und der Umfang der vom Verfahren zu verwendenden Zahntypen festgelegt. Die Scandaten **D** können beispielsweise unmittelbar von der Modalität **1** bzw. deren Steuereinrichtung **2** über den Datenbus **3** dem Computer **10** zugeführt werden. Es kann sich aber auch um Scandaten **D** handeln, die bereits vor einiger Zeit aufgenommen und in einem Massenspeicher **4** hinterlegt wurden. Optional kann eine interaktive Bearbeitung der Scandaten **D** durch den Bediener stattfinden, bei dem der Bediener Strukturen in den Scandaten **D** markiert, die den Individualisierungsvorgang der Zahnmodelle **M** unterstützen. Insbesondere können Präparationslinien **LP,** Segmentierungslinien **LS,** anatomische Landmarken **AL,** Kontaktpunkte und Markierungen einzelner Zähne - interaktiv oder mit algorithmischer Unterstützung - bestimmt werden. **Fig. 4** zeigt hierzu eine schematische zweidimensionale Darstellung von Scandaten **D** mit Antagonisten-Daten, markierten Präparationslinien **LP,** markierten Segmentierungslinien **LS** und markierten anatomischen Landmarken **AL.**

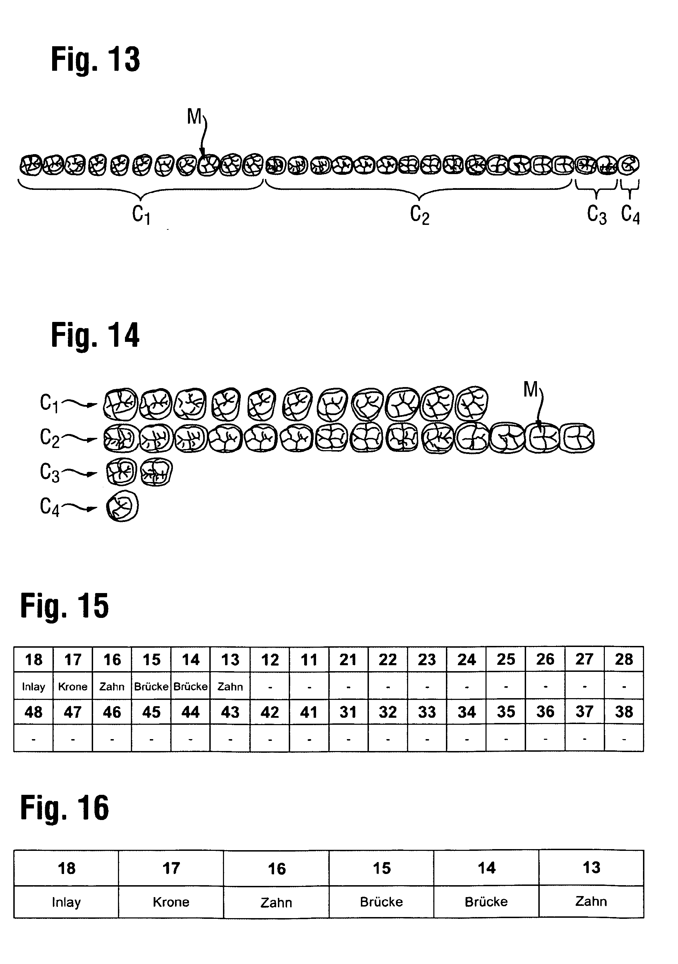

Der Umfang der vom Verfahren zu verwendenden Zahnmodelle **M** wird vorzugsweise durch die Angabe einer Menge unterschiedlicher Zahntypen festgelegt. Wegen der besseren Anschaulichkeit für den Bediener kann diese Angabe mittels einer grafischen Benutzeroberfläche und einem Zahnschema erfolgen, das beispielhaft in **Fig. 15** dargestellt ist. Die dazugehörigen Mittelwert-Zahnmodelle **M** der Zahntypen 18 bis 48 zeigt die **Fig. 3****.** Vorzugsweise sollte der Umfang der zu verwendenden Zahntypen dem Umfang der Zahntypen der gescannten Zähne entsprechen. Nach der Festlegung der gewünschten Zahntypen ist es von Vorteil, das Zahnschema in einer reduzierten Form dem Bediener anzuzeigen (z.B. wie in **Fig. 16****).** Im Folgenden wird davon ausgegangen, dass der Bediener mehrere Zahntypen ausgewählt hat. In diesem Fall ist die Verwendung derjenigen Variante des erfindungsgemäßen Verfahrens sinnvoll, bei der Gruppen von Zahnmodellen **M** individualisiert werden.

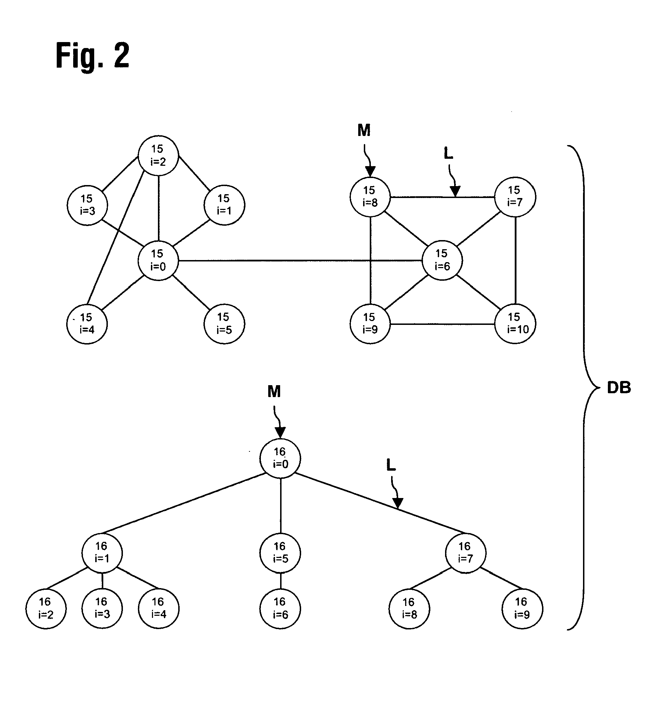

Ergänzend kann auch die Struktur der Verlinkung **L** der verwendeten Modelldatenbank **DB** (wie in **Fig. 2****)** und/oder eine lineare Sortierung **P** der Zahnmodelle der Modelldatenbank **DB** (wie in **Fig. 13** und **Fig. 14****)** zur Auswahl einer Auswahleinheit zugeführt und mit Hilfe eines Auswahlsignals gewünschte Zahnmodelle **M** selektiert werden, die dann vom Verfahren verwendet werden. Durch dieses Vorgehen kann der Bediener gewünschte Untergruppen von Zahnmodellen **M** eines Zahntyps auswählen, um beispielsweise Zahnmodelle **M** mit besonderen morphologischen Eigenschaften dem Verfahren zuzuführen. Dieses Vorgehen kann insbesondere für die Berechnungen von Zahnrestaurationen im Frontzahnbereich von Vorteil sein. Die Prozesse des ersten Verfahrensschritts werden mittels einer Auswahleinheit **14** durchgeführt, welche hier in Form eines Softwaremoduls auf dem Prozessor **13** des Computers **10** realisiert ist.

**Figur 2** zeigt hierzu eine sehr vereinfachte schematische Darstellung einer Modelldatenbank **DB** mit verlinkten Zahnmodellen **M** für zwei Zahntypen 15, 16. Den Kreisen entsprechen Zahnmodelle **M,** die mit optionalen geometrischen Transformationen versehen sind. In den Kreisen - auch Knoten der Modelldatenbank **DB** genannt - sind die Zahntypen und die Auswahlindices für die dazugehörige Modellklasse abgebildet. Aus Anschaulichkeitsgründen wird nur eine geringe Anzahl von Zahnmodellen **M** dargestellt und die Verlinkung **L** (netzförmig für Zahntyp 15, hierarchisch für Zahntyp 16) der Zahnmodelle **M** ist besonders einfach gehalten.

Die Bestimmung der optimalen Zahnmodelle für die gewünschten Zahntypen erfolgt bei dem beschriebenen Ausführungsbeispiel durch einen Prozess mit mehreren Iterationsschritten **S,** wobei beginnend mit Start-Zahnmodellen der gewünschten Zahntypen bei jedem Iterationsschritt **S** eine Gruppe von Zahnmodellen hinsichtlich eines Qualitätswerts getestet wird. Jeder Iterationsschritt umfasst dabei mehrere Verfahrensschritte **II**, **III, IV, V**, die in Form einer Schleife **S** durchlaufen werden. Dieser Optimierungsprozess findet in der Optimierungseinheit **15** des Zahnrestaurationsermittlungssystems **5** statt, welche hier in Form eines Softwaremoduls auf dem Prozessor **13** des Computers **10** realisiert ist.

Vor dem ersten Durchlauf der Schleife **S** werden die Start-Zahnmodelle für die gewünschten Zahntypen bestimmt und in den Scandaten platziert. Vorzugsweise sind die Start-Zahnmodelle in der Modelldatenbank festgelegt und sind besonders bevorzugt Mittelwert-Zahnmodelle der gewünschten Zahntypen. Alternativ können Start-Zahnmodelle gewünschter Zahntypen auch aus einer geometrischen Analyse der Scandaten bestimmt werden. Eine Möglichkeit ist die automatische oder manuelle Vermessung von Zähnen und/oder von Restzahnsubstanzen in den Scandaten, um dann Start-Zahnmodelle aus der Modelldatenbank mit möglichst ähnlichen geometrischen Maßen auszuwählen. Typische geometrische Maße sind beispielsweise Zahnbreite, Zahntiefe, Höckerabstände und Schneidekantendicke. Nach der Auswahl der Start-Zahnmodelle sollten diese vor dem ersten Schleifendurchlauf relativ zu den Scandaten grob platziert werden, d.h. es wird eine Vorpositionierung der Start-Zahnmodelle vorgenommen. Bei radiologischen Scandaten (z.B. DVT-Scandaten) sind in der Regel Richtungsbezeichnungen (anterior, posterior, etc.) des Datensatzes bekannt und das Aufnahmevolumen umfasst normalerweise den Mundraum mit angrenzenden Strukturen. Ähnlich ist die Situation bei optischen Kieferscans. Auch dort werden in der Regel Richtungskonventionen für die Scandaten eingehalten. Die Okklusionsflächen der Zähne zeigen in eine definierte Achsrichtung und die parabelförmigen Zahnbögen sind zu einer anderen definierten Achsrichtung hin geöffnet. Bei intraoralen optischen Scans ist die Situation geringfügig unterschiedlich. Auch hier zeigen die Okklusionsflächen der Zähne in eine definierte Achsrichtung und für die Enden des gemessenen Teils eines Zahnbogens sind Richtungsbezeichnungen (z.B. mesial, distal) bekannt. Aufgrund dieser impliziten Richtungsinformationen und der bekannten Abmessungen der Scandaten können die Start-Zahnmodelle zum Verfahrensstart relativ zu den Scandaten platziert werden.

Die Schleife **S** beginnt mit dem Verfahrensschritt **II,** bei dem die aktuell zu testende Gruppe von Zahnmodellen und zumindest eine weitere Gruppe von Zahnmodellen aus einer Modelldatenbank mittels einer Ladeeinheit **16** geladen werden, welche hier in Form eines Softwaremoduls auf dem Prozessor **13** des Computers **10** realisiert ist. Hierzu weist der Computer **10** einen Speicher **12** mit einer Modelldatenbank **DB** auf. Die **Fig. 2** stellt exemplarisch eine mögliche Verlinkung **L** von Zahnmodellen **M** für die Zahntypen 15 und 16 einer Modelldatenbank **DB** dar.

Im Verfahrensschritt **III** werden dann Gruppen von Zahnmodellen durch Variation von Modellparametern an die Scandaten angepasst. Dieser Individualisierungsprozess findet in der Individualisierungseinheit **17** statt, welche hier in Form eines Softwaremoduls auf dem Prozessor **13** des Computers **10** realisiert ist. Die Individualisierung von Zahnmodellen wird vorzugsweise durch die Lösung einer Optimierungsaufgabe durchgeführt, bei der sich der Optimierungswert aus einer Anzahl von Optimierungsteilwerten ergibt, die gewünschten Optimierungskriterien entsprechen. Die einzelnen Optimierungsteilwerte werden nach deren Berechnung zu einem einzigen Optimierungswert zusammengefasst. Vorzugsweise geschieht dies durch die Berechnung einer gewichteten Summe der Optimierungsteilwerte. Die Gewichtungsfaktoren ermöglichen dabei eine Steuerung des Einflusses der einzelnen Optimierungskriterien. So kann beispielsweise ein hoher Gewichtungsfaktor für die Anpassung der Zahnmodelle an die Gegenbezahnung in Kombination mit einem niedrigen Gewichtungsfaktor für die Anpassung an die Nachbarzähne zu optimalen Zahnmodellen führen, die sehr gut an die Gegenbezahnung angepasst sind, dafür aber keine hundertprozentige Kontaktsituation zu den Nachbarzähnen aufweisen. Vorzugsweise werden die Gewichtungsfaktoren so gewählt, dass alle Optimierungskriterien gleichermaßen gut eingehalten werden. Die Lösung der im Allgemeinen nichtlinearen Optimierungsaufgabe kann durch dem Fachmann bekannte Verfahren erfolgen. Eine einfache Möglichkeit ist die Bestimmung des minimalen Optimierungswertes durch Gradienten-Abstiegsverfahren.

Ein wichtiger Optimierungsteilwert beschreibt die Anpassung eines Zahnmodells an die dazugehörige Zahn- und/oder Restzahnsubstanz in den Scandaten. Diese Zielstruktur wird vorzugsweise durch in den Scandaten bestimmte Präparations- und/oder Segmentierungslinien definiert. Die zur Bestimmung der Grenzlinien notwendigen automatischen und/oder interaktiven Verfahren sind dem Fachmann bekannt. Optional kann auch eine direkte Markierung von Zahn- und/oder Restzahnsubstanz durch den Bediener stattfinden. Die Berechnung des Optimierungsteilwerts erfolgt vorzugsweise durch die Bildung der Summe von Quadraten von Entfernungswerten der Zahnmodell-Vertices zur Zielstruktur. Als Entfernungswerte können die minimalen Entfernungen der Zahnmodell-Vertices zur Zielstruktur, aber auch die Entfernungen entlang der Flächennormalen der Zahnmodell-Vertices zur Zielstruktur verwendet werden.

Ein weiterer wichtiger Optimierungsteilwert beschreibt bei dem vorliegenden Ausführungsbeispiel den Kontakt eines Zahnmodells zur Gegenbezahnung. Dabei wird die Gegenbezahnung direkt oder indirekt durch ein Bissregistrat vermessen. In beiden Fällen erhält man so genannte Antagonisten-Daten. Zur Beschreibung der Kontaktsituation eines Zahnmodells zu einem dazugehörigen Antagonisten wird die Verwendung von vorzeichenbehafteten Entfernungswerten vorgeschlagen. Ein negatives Vorzeichen für einen Entfernungswert eines Zahnmodell-Vertex ist dann gegeben, wenn er innerhalb des Antagonisten liegt, ansonsten erhält er ein positives Vorzeichen. Bestimmt man nun das Minimum der Entfernungswerte, so erhält man für ein Zahnmodell einen einzigen vorzeichenbehafteten Kontakt-Enfernungswert. Ist er positiv, so liegt keine Durchdringung vor, ist er Null, so existieren ein oder mehrere Kontaktpunkte, ist er negativ, so liegt eine Durchdringung vor. Der Optimierungsteilwert ist dann vorzugsweise das Quadrat des Kontakt-Entfernungswerts. Optional kann der Bediener auch noch gewünschte Kontaktpunkte und/oder -flächen zum Antagonisten interaktiv festlegen. Die Entfernungen der dazugehörigen Scanelemente zum Zahnmodell gehen dann zusätzlich in die Berechnung des Optimierungsteilwerts ein. Vollkommen analog zu der vorgeschlagenen Berechnungsvorschrift zur Beschreibung des Kontaktes zur Gegenbezahnung kann ein Optimierungsteilwert für den Kontakt der Zahnmodelle untereinander und/oder zur Nachbarbezahnung berechnet werden.

Die Berechnung eines Optimierungsteilwerts für die Anpassung eines Zahnmodells an Präparations- und/oder Segmentierungslinien kann auch durch die Verwendung vorzeichenbehafteter Entfernungswerte erfolgen. Dazu werden vorzugsweise Entfernungswerte der Grenzlinien-Vertices zum Zahnmodell bestimmt. Ein negatives Vorzeichen zeigt an, dass ein Vertex innerhalb des Zahnmodells liegt, ein positives Vorzeichen zeigt hingegen an, dass ein Vertex außerhalb liegt. Als Grenzlinien-Entfernungswert definiert man vorzugsweise das Maximum der positiven Entfernungswerte und bildet dessen Quadrat, um den dazugehörigen Optimierungsteilwert zu erhalten.

Ein weiterer Optimierungsteilwert beschreibt die Anpassung eines Zahnmodells an anatomische Landmarken in den Scandaten. Die Bestimmung dieser Landmarken kann automatisch und/oder interaktiv erfolgen und ist dem Fachmann bekannt. Zur Berechnung des Optimierungsteilwerts werden in einer bevorzugten Variante die Entfernungen der anatomischen Landmarken in den Scandaten zu den korrespondierenden Landmarken des Zahnmodells berechnet. Der Optimierungsteilwert ergibt sich dann über die Summe der Quadrate dieser Entfernungswerte.

Ebenfalls nach dem Prinzip von vorzeichenbehafteten Entfernungswerten ist eine einfache robuste Berechnung eines Optimierungsteilwerts für die mechanische Stabilität der zu einem Zahnmodell gehörenden Zahnrestauration möglich. Dazu wird vorzugsweise aus den Scandaten und der zu einem Zahnmodell gehörenden Präparationslinie ein Hilfsobjekt konstruiert, welches die minimale Materialstärke für die Zahnrestauration definiert. Eine Durchdringung des Zahnmodells mit diesem Minimalobjekt soll vermieden werden. D.h. bei einem positiven Kontakt-Entfernungswert eines Zahnmodells zu dem dazugehörigen Minimalobjekt ist der Optimierungsteilwert Null, ansonsten das Quadrat des Kontakt-Entfernungswerts.

Neben den beispielhaft aufgeführten funktionalen Optimierungsteilwerten kann auch ein ästhetischer Optimierungsteilwert für ein Zahnmodell, vorzugsweise für ein Frontzahn-Zahnmodell, bestimmt werden. Zu dessen Berechnung wird vorzugsweise automatisch und/oder interaktiv eine gewünschte Form (rechteckig, dreieckig, quadratisch, schaufelförmig etc.) des Zahnmodells ausgewählt und ein Optimierungsteilwert berechnet, der die Abweichung des Zahnmodells von der gewünschten Form beschreibt. Eine bevorzugte Berechnungsmöglichkeit für die Abweichung besteht in der Bestimmung der Kontur des Zahnmodells in lingual-oraler Richtung und anschließender Anpassung dieser Kontur durch Translation, Rotation sowie Skalierung an die bevorzugte Form. Dann werden für die Konturpunkte der bevorzugten Form die minimalen Entfernungen zu der angepassten Kontur des Zahnmodells bestimmt. Der zu berechnende Optimierungsteilwert ist dann vorzugsweise die Summe der Quadrate dieser Entfernungswerte.

Eine Kopplung von Zahnmodellen wird vorzugsweise durch Optimierungsteilwerte berücksichtigt, welche die räumlichen Relationen der Lagen und/oder Formen der gekoppelten Zahnmodelle beschreiben. Der Optimierungsteilwert für die räumlichen Relationen der Lagen kann durch räumliche Relationen von anatomischen Landmarken gekoppelter Zahnmodelle berechnet werden. Die Bestimmung geeigneter Landmarken findet vorzugsweise im Rahmen des Aufbaus einer Modelldatenbank statt und kann interaktiv und/oder algorithmisch erfolgen. Eine vorgeschlagene Berechnungsart für den Optimierungsteilwert basiert darauf, dass anatomische Landmarken gekoppelter Zahnmodelle auf parametrisierten dreidimensionalen Kurven liegen sollen. Als Optimierungswert wird vorzugsweise die Summe der Quadrate der Entfernungswerte von den Landmarken zu den Kurven gebildet. Beispielsweise sollen die Schneidekanten und Höcker der oberen Zähne auf einer Speeschen Kurve liegen. Ebenso wichtig für die Kopplung von Zahnmodellen sind die relativen Relationen der Formen der Zahnmodelle untereinander. Es wird vorgeschlagen, dass zumindest Teile einer Modelldatenbank auf der Basis einer repräsentativen Menge von Probanden-Scandaten aufgebaut werden. Zu einem Zahnmodell eines Zahntyps korrespondieren dann diejenigen Zahnmodelle der anderen Zahntypen, die vom gleichen Probanden stammen. Der Optimierungsteilwert für die Kopplung der Formen von Zahnmodellen ergibt sich vorzugsweise aus der Berechnung der Abweichungen von den Korrespondenzen der Zahnmodelle. Dazu werden für jedes zu individualisierende Zahnmodell zuerst die korrespondierenden Zahnmodelle aus der Modelldatenbank bestimmt, deren Zahntypen in der Kopplungsgruppe enthalten sind. Danach findet eine Berechnung von morphologischen Abweichungswerten der bestimmten korrespondierenden Zahnmodelle zu den dazugehörigen individualisierten Zahnmodellen statt. Die Berechnung eines morphologischen Abweichungswerts kann einfach und robust über die Summe von Entfernungswert-Quadraten der Vertices von zwei zu vergleichenden Zahnmodellen erfolgen, nachdem die beiden Zahnmodelle durch Translation und Rotation möglichst optimal zur Deckung gebracht wurden. Der so bestimmte Abweichungswert wird im Folgenden als Korrespondenz-Abweichungswert bezeichnet. Der Optimierungsteilwert für die Kopplung der Formen der Zahnmodelle ergibt sich dann als die Summe der Quadrate der Korrespondenz-Abweichungswerte aller zu individualisierenden Zahnmodelle untereinander.

Im Verfahrensschritt **IV** erfolgt innerhalb einer Qualitätsermittlungseinheit **18** die Berechnung von Qualitätswerten für individualisierte Gruppen von Zahnmodellen, welche hier in Form eines Softwaremoduls auf dem Prozessor **13** des Computers **10** realisiert ist. Ein Qualitätswert von 100% sollte für die Individualisierung einer Gruppe von Zahnmodellen verwendet werden, bei der alle Optimierungskriterien vollständig erfüllt sind. Geringere Prozentwerte beschreiben eine Individualisierung, bei der einzelne Optimierungskriterien nur teilweise oder überhaupt nicht erfüllt werden. Vorzugsweise wird der Qualitätswert aus dem Optimierungswert der dazugehörigen Gruppe von Zahnmodellen durch eine Umrechung bestimmt. Der minimal mögliche Optimierungswert entspricht in diesem Fall einem Qualitätswert von 100% und der maximal mögliche Optimierungswert dem Qualitätswert 0%.

Am Ende der Schleife **S** wird im Verfahrensschritt **V** entschieden, ob auf Basis der berechneten Qualitätswerte eine neue zu testende Gruppe von Zahnmodellen der gewünschten Zahntypen aus der Modelldatenbank für den nächsten Iterationsschritt **S** ausgewählt wird oder ob die Iteration abgebrochen wird. Dies hängt davon ab, ob ein vorgegebenes Qualitätskriteriums erreicht wurde. Ein solches Qualitätskriterium kann z.B. sein, dass der Qualitätswert der aktuell zu testenden Gruppe von Zahnmodellen einen Schwellenwert überschritten hat oder eine vorgegebene Anzahl von Iterationsschritten erreicht wurde.

Soll ein weiterer Iterationsschritt erfolgen, kann eine neue zu testende Gruppe von Zahnmodellen dadurch bestimmt werden, dass der dazugehörige Qualitätswert besser als der Qualitätswert der aktuell zu testenden Gruppe von Zahnmodellen ist. Dazu können nacheinander die Qualitätswerte von Gruppen von Zahnmodellen berechnet werden, wobei sich die Gruppen durch Variation der aktuell zu testenden Gruppe ergeben, derart, dass zumindest ein Zahnmodell der aktuell zu testenden Gruppe durch ein mit ihm verlinktes Zahnmodell ersetzt wird, bis man einen besseren Qualitätswert gefunden hat. Findet man keinen besseren Qualitätswert oder ist der Qualitätswert der aktuell zu testenden Gruppe von Zahnmodellen ausreichend, dann wird ebenfalls die Iteration **S** abgebrochen. Insofern kann auch die Tatsache, dass kein weiteres Zahnmodell beziehungsweise weitere Gruppe von Zahnmodellen mit einem besseren Qualitätswert gefunden werden kann, als Erreichung eines Qualitätskriteriums zum Abbruch der Iteration gesehen werden.

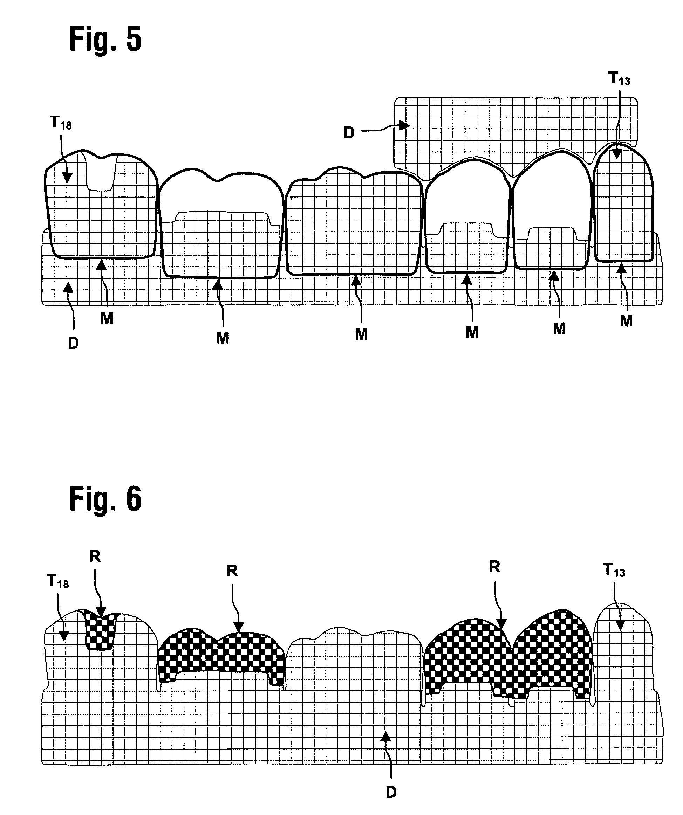

Im abschließenden Verfahrensschritt **VI** wird schließlich aus den optimalen Zahnmodellen **M** und Scandaten **D** zumindest eine Zahnrestauration **R** durch Standardverfahren bestimmt, die dem Fachmann bekannt sind. Prinzipiell werden dazu die Präparationslinien der Scandaten **D** in geeigneter Weise möglichst stetig und glatt mit den optimalen Zahnmodellen **M** verbunden und dann die Kavitäten als untere Begrenzungen hinzugefügt. Als Ergebnis erhält man für die Zahnrestaurationen **R** dreidimensionale Modelle von Schleifkörpern, die maschinell hergestellt werden können. Man kann aber auch eine Zahnrestauration **R** mehrteilig gestalten, indem zuerst ein Trägergerüst und danach eine Oberkonstruktion mit der Kaufläche bestimmt wird. Zur Steigerung der Präzision der zu bestimmenden Zahnrestaurationen **R** kann man vor deren Konstruktion eine Feinanpassung der optimalen Zahnmodelle **M** untereinander und/oder an die Scandaten **D** durchführen. Von Vorteil ist die Anwendung lokal beschränkter Deformationstransformationen auf die optimalen Zahnmodelle **M,** welche eine Feinanpassung der Zahnmodelle **M** untereinander sowie an die Zähne, Restzahnsubstanzen, Präparationslinien, Segmentierungslinien, Bissregistrate, Kontaktpunkte, etc. mit möglichst geringen Verschiebungswerten vornehmen und dabei keine Kanten oder Faltungen erzeugen. Die Berechnungen des Verfahrensschrittes **VI** finden innerhalb einer Restaurationseinheit **19** statt, welche hier in Form eines Softwaremoduls auf dem Prozessor **13** des Computers **10** realisiert ist. In der **Fig. 5** sind exemplarisch Scandaten **D** mit optimalen Zahnmodellen **M** für typische Inlay-, Kronen- und Brückenpräparationen dargestellt. **Fig. 6** zeigt die dazugehörigen Zahnrestaurationen **R.**

Anschließend erfolgt dann die Herstellung oder Auswahl zumindest eines Zahnrestaurationsteils basierend auf den ermittelten Zahnrestaurationen.

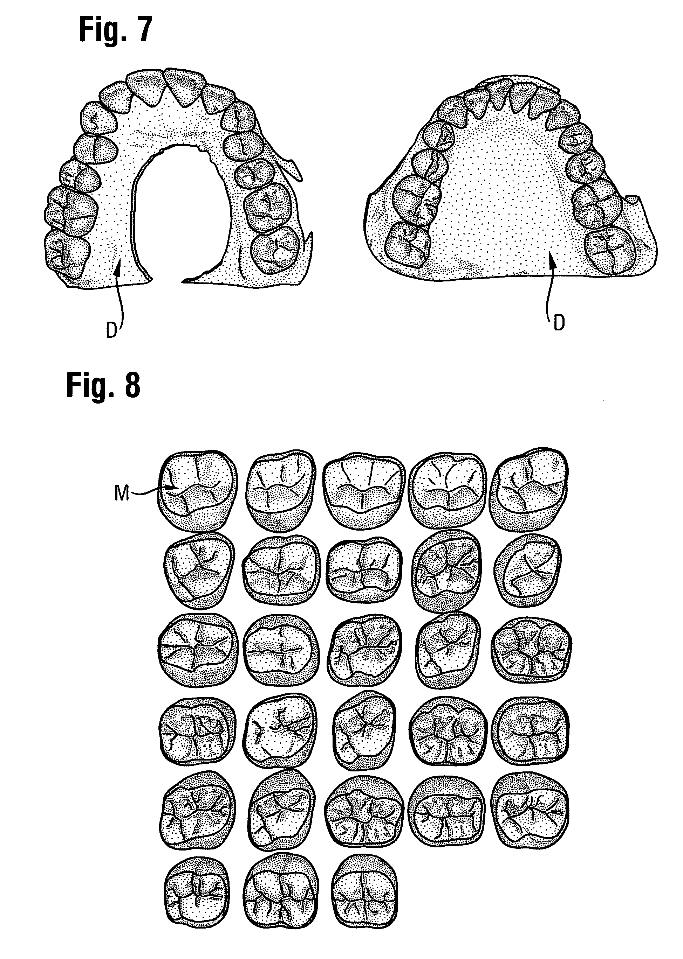

Ein Ausführungsbeispiel eines erfindungsgemäßen Verfahrens zum Aufbau von zumindest Teilen einer Modelldatenbank ist dadurch gekennzeichnet, dass eine Analyse einer repräsentativen Menge von Scandaten **D** defektfreier Ober- und Unterkiefer durchgeführt wird. Vorgeschlagen wird die Verwendung von Kiefer-Gipsabformungen, welche kostengünstig und präzise optisch eingescannt werden können. **Fig. 7** zeigt auf diese Weise erzeugte Scandaten **D.** Für die Analyse der Formvariationen der Einzelzähne ist es vorteilhaft, die Scandaten **D** in einem ersten Schritt des Datenbankaufbaus zu segmentieren, wobei diese Segmentierung vorzugsweise interaktiv mit Hilfe einer grafischen Benutzeroberfläche durchgeführt werden kann. Als Ergebnis der Segmentierung von Kiefer-Scandaten **D** erhält man für jeden Zahntyp (hier Zahnnummer) eine Menge von Zahn-Scandatensätzen, welche zum Aufbau zumindest eines Teils einer Modelldatenbank verwendet werden kann.

Die Konstruktion von Zahnmodellen **M** einer Modellklasse findet im zweiten Schritt des Datenbankaufbaus statt. Je nach gewünschter räumlicher Auflösung wird für jeden Zahn-Scandatensatz ein Zahnmodell **M** konstruiert. Dazu kann man zuerst ein Zahnmodell **M** konstruieren, welches aus einer triangulierten Oberfläche in Form eines Quaders mit den Abmessungen des Zahn-Scandatensatzes besteht, wobei sich die Triangulierung einer gewünschten Auflösung durch sukzessive Unterteilung der Seitenflächen des Quaders ergibt. Danach findet eine Anpassung des initial konstruierten Zahnmodells an den Zahn-Scandatensatz statt, beispielsweise durch die Minimierung der Summe der Quadrate der Entfernungswerte der Zahnmodell-Vertices zur Zahnoberfläche in dem Zahn-Scandatensatz. **Fig. 8** zeigt auf diese Weise konstruierte Zahnmodelle **M einer** Modellklasse.

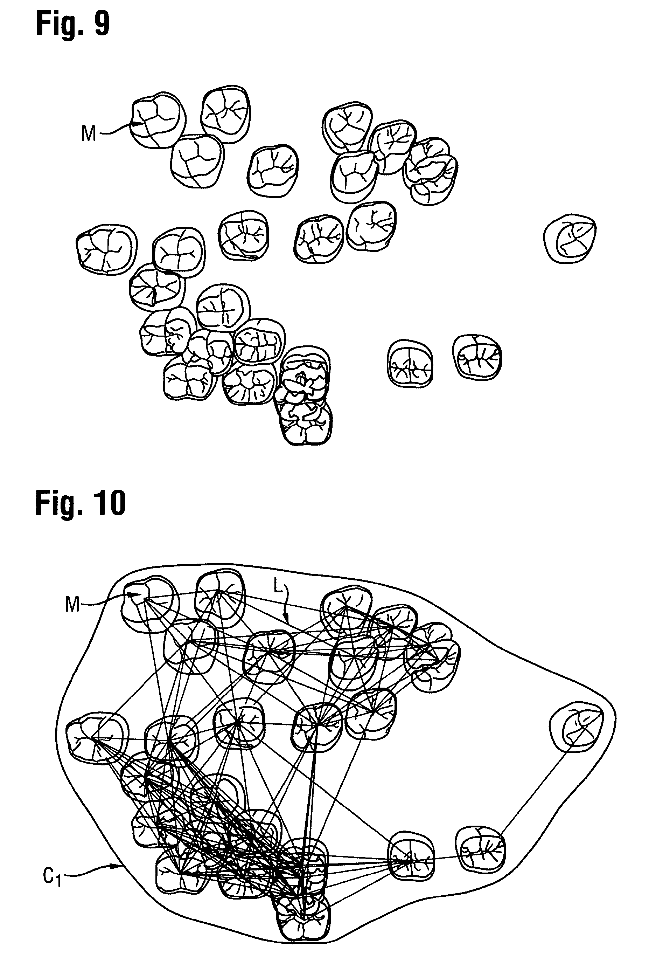

Anschließend findet im dritten Schritt des Datenbankaufbaus eine Analyse der morphologischen Abweichungen der Zahnmodelle **M** einer Modellklasse untereinander statt, indem für jedes mögliche Paar von Zahnmodellen **M** ein Abweichungswert berechnet wird. Dazu werden die beiden Zahnmodelle **M** eines Paars vorzugsweise durch eine optimale Translation und Rotation derart zur Deckung gebracht, dass die Summe der Quadrate von Entfernungswerten der Zahnmodell-Vertices minimal ist. Als Entfernungswert eines Zahnmodell-Vertex des ersten Zahnmodells **M** des Paares kann der minimale Abstand des Vertex zur Oberfläche des zweiten Zahnmodells **M** des Paares verwendet werden. Der nach der Anpassung noch verbleibende mittlere Entfernungswert der Zahnmodell-Vertices kann als Abweichungswert für das Zahnmodell-Paar verwendet werden. Eine Visualisierung der Ergebnisse erfolgt vorzugsweise durch eine zweidimensionale Aufstellung der Zahnmodelle **M,** wie in der **Fig. 9** gezeigt. Diese Aufstellung ist dadurch definiert, dass die Entfernungen der Zahnmodelle **M** untereinander bis auf einen gemeinsamen Skalierungsfaktor möglichst den dazugehörigen Abweichungswerten der Zahnmodelle **M** entsprechen.

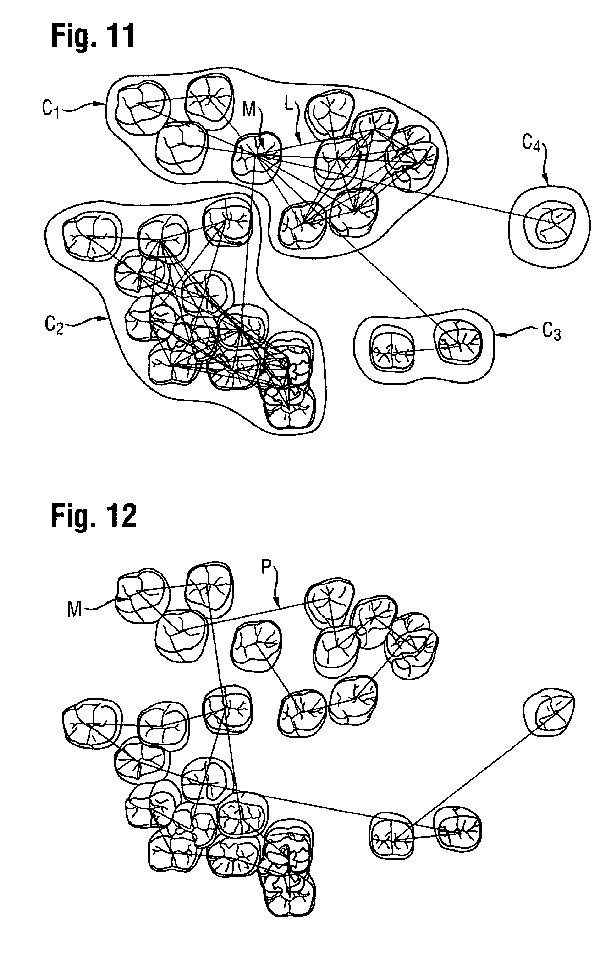

Im vierten und fünften Schritt des Datenbankaufbaus findet eine Verlinkung **L** der Zahnmodelle **M** einer Modellklasse statt. Dazu werden Cluster morphologisch ähnlicher Zahnmodelle **M** konstruiert, wobei die Konstruktionsvorschrift erfindungsgemäß auf morphologischen Abweichungswerten der Zahnmodelle **M** untereinander basiert. Zur Konstruktion wird vorzugsweise zuerst ein Schwellenwert für die Abweichungswerte der Zahnmodelle **M** vorgegeben und diejenigen Zahnmodelle **M** verlinkt, deren Abweichungswerte unterhalb dieser Schwelle liegen. Ein Cluster ist dann eine zusammenhängende Menge untereinander verlinkter morphologisch ähnlicher Zahnmodelle **M.** Die **Fig. 10** und **Fig. 11** zeigen Beispiele für eine Menge von Zahnmodellen **M** mit einem Cluster **C₁** (gemäß **Fig. 10****)** bzw. vier Clustern **C₁**, **C₂**, **C₃, C₄** (gemäß **Fig. 11****).** Ein grober Richtwert für den Schwellenwert ergibt sich vorzugsweise aus der Forderung, dass der Umfang des größten Clusters ungefähr der Hälfte der Gesamtanzahl der Zahnmodelle **M** der Modellklasse entspricht. Anschließend findet eine Bestimmung von Clustermittelwert-Zahnmodellen **M** statt, indem für jeden Cluster dasjenige Zahnmodell **M** bestimmt wird, dessen Summe der Quadrate der Abweichungswerte zu den anderen Zahnmodellen **M** des Clusters minimal ist. Von besonderer Bedeutung ist dasjenige Clustermittelwert-Zahnmodell **M,** das eine minimale Abweichung - wieder definiert über die Summe der Quadrate der Abweichungswerte - zu allen vorhandenen Zahnmodellen **M** der Modellklasse besitzt. Dieses Zahnmodell **M** wird dann als Mittelwert-Zahnmodell **M** der Modellklasse angesehen. Damit die Zahnmodelle **M** vollständig zusammenhängend verlinkt sind, werden abschließend Verlinkungen **L** vom Mittelwert-Zahnmodell **M** zu allen Clustermittelwert-Zahnmodellen **M** hinzugefügt.

Im abschließenden sechsten Schritt des Datenbankaufbaus werden noch geometrische Transformationen zu den Zahnmodellen hinzugefügt, vorzugsweise dadurch definiert, dass die Form eines Zahnmodells in alle mit ihm verlinkten Zahnmodelle des gleichen Clusters kontinuierlich überführt werden kann. Dies wird vorzugsweise durch die Konstruktion einer parametrisierten Transformation für jedes Zahnmodell erreicht. Der erste Schritt besteht dabei in einer Unterteilung der Bounding-Box eines Zahnmodells in gleichgroße achsenparallele Zellen. Translationsvektoren der Zellenknoten definieren dann eine trilineare Transformation der Zahnmodell-Vertices. Dieser Ansatz wird im Detail in der Promotionsarbeit "Individualisierung von digitalen anatomischen Modellen durch Computertomographie" (Tank M., 2002, Institut für Rechtsmedizin der Universität Heidelberg) beschrieben und dort auch als Zellenverfahren bezeichnet. Als Ergebnis erhält man eine Transformation, bei der die Transformationsparameter hierarchisch nach ihrer Wichtigkeit geordnet sind. Die Transformationsparameter für eine Verlinkung zweier Zahnmodelle ergeben sich aus der Forderung, dass die Summe der Quadrate der Entfernungswerte des transformierten Zahnmodells zu dem verlinkten Zahnmodell minimal ist und das lokale Flächeneigenschaften möglichst erhalten bleiben. Eine Linearkombination von auf dieser Weise erhaltenen Transformationsparametern ergibt morphologisch sinnvolle Variationen eines Zahnmodells, da ja nur diejenigen verlinkten Zahnmodelle als Transformationsziele verwendet werden, die aus dem gleichen Cluster stammen und somit morphologisch ähnlich sind. Die Formparameter eines Zahnmodells umfassen dann die auf diese Weise definierten Linearfaktoren.

Neben dem Aufbau von zumindest Teilen einer Modelldatenbank auf der Basis von Scandaten natürlicher oraler Strukturen können aber auch Scandaten von künstlichen oralen Strukturen verwendet werden. Das Aufbauverfahren für die dazugehörige Modelldatenbank entspricht dem Aufbauverfahren für natürliche orale Strukturen und wird dabei vollkommen analog durchgeführt. Es können dann der erste und/oder letzte Schritt des Datenbankaufbaus entfallen. Der erste Schritt des Datenbankaufbaus, d.h. die Segmentierung der Scandaten, ist in der Regel nicht nötig, da die künstlichen oralen Strukturen (beispielsweise künstliche Zähne wie Prothesenzähne oder Zahnrohlinge) physisch separiert vorliegen und einzeln scannbar sind. Aufgrund der optionalen Forderung, dass keine geometrischen Transformationen für die Zahnmodelle verwendet werden sollen, kann auch der letzte Schritt des Datenbankaufbaus entfallen. Insbesondere für die interaktive Auswahl von Zahnmodellen **M** ist eine lineare Sortierung **P** der Zahnmodelle **M** einer Modellklasse aufgrund morphologischer Ähnlichkeiten vorteilhaft. Zum Aufbau einer Sortierung kann man die Sortierung mit dem Mittelwert-Zahnmodell **M** beginnen und fügt iterativ weitere Zahnmodelle **M** der Modellklasse an. Das jeweils zuletzt angefügte Zahnmodell **M** wird dabei als aktuelles Zahnmodell **M** bezeichnet. Das Sortierkriterium kann dadurch definiert sein, dass immer dasjenige Zahnmodell **M** hinzugefügt wird, welches den kleinsten Abweichungswert zu dem aktuellen Zahnmodell **M** besitzt und innerhalb des dazugehörigen Clusters liegt. Sind alle Zahnmodelle **M** eines Clusters angehängt, wird das Verfahren für denjenigen Cluster fortgesetzt, dessen Clustermittelwert-Zahnmodell **M** den geringsten Abweichungswert zum aktuellen Zahnmodell **M** besitzt. **Fig. 12** zeigt einen so erhaltenen Sortierpfad **P** für eine Modellklasse. Zwei übersichtlichere Darstellungsweisen für die Sortierung **P** zeigen die **Fig. 13** und **Fig. 14****.** Gemäß **Fig. 13** werden die Zahnmodelle **M** eindimensional aufgestellt und der Bediener kann beispielsweise mit Hilfe von Mauseingaben einer grafischen Benutzeroberfläche ein oder mehrere Zahnmodelle **M** aus der Sortierung **P** auswählen. Bei der zweidimensionalen kompakteren Aufstellung gemäß **Fig. 14** wird die Sortierung **P** zeilenweise umgebrochen, vorzugsweise definiert durch die Clusterzugehörigkeit **C₁, C₂, C₃, C₄.** Hier kann der Bediener ebenfalls mit Hilfe von Mauseingaben einer grafischen Benutzeroberfläche ein oder mehrere Zahnmodelle **M** auswählen.

Es wird an dieser Stelle noch einmal ausdrücklich darauf hingewiesen, dass es sich bei den in den Figuren dargestellten Systemarchitekturen und Prozessen nur um Ausführungsbeispiele handelt, die vom Fachmann ohne weiteres im Detail verändert werden können. Insbesondere kann die Steuereinrichtung, sofern sie beispielsweise mit einer entsprechenden Konsole eingerichtet ist, auch alle entsprechenden Komponenten des Computers aufweisen, um dort unmittelbar die Messdatenverarbeitung nach dem erfindungsgemäßen Verfahren durchzuführen. In diesem Fall bildet folglich die Steuereinrichtung selbst das erfindungsgemäße Zahnrestaurationsermittlungssystem, und eine weitere Workstation bzw. ein separater Computer ist nicht erforderlich. Im Übrigen ist es auch nicht zwingend notwendig, dass die verschiedenen Komponenten eines erfindungsgemäßen Zahnrestaurationsermittlungssystems auf einem Prozessor bzw. in einem Computer o.Ä. realisiert sind, sondern die verschiedenen Komponenten können auch auf mehrere Prozessoren bzw. untereinander vernetzten Computern verteilt sein. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließen die Begriffe "Vorrichtung", "Einrichtung", "Einheit", "Modul" etc. nicht aus, dass diese aus mehreren Komponenten bestehen, die gegebenenfalls auch räumlich verteilt sein können.

Es bietet sich im Übrigen an, bestehende Zahnrestaurationsermittlungssysteme, in welchen bereits bekannte Nachverarbeitungsprozesse implementiert sind, mit einer erfindungsgemäßen Prozesssteuereinheit nachzurüsten, um auch diese Anlagen gemäß dem vorstehend beschriebenen erfindungsgemäßen Verfahren zu nutzen. In vielen Fällen reicht ggf. auch ein Update der Steuerungssoftware mit geeigneten Steuerungs-Softwaremodulen aus.

## Patentansprüche

1. Verfahren zur Ermittlung von virtuellen Zahnrestaurationen auf der Basis von Scandaten (D) oraler Strukturen, bei dem
- eine Modelldatenbank (DB) verwendet wird, die für verschiedene Zahntypen jeweils eine Anzahl von parametrisierten Zahnmodellen (M) enthält, wobei die Parametrisierung anhand von Modellparametern erfolgt, welche Lage- und/oder Formparameter umfassen, und wobei jedes Zahnmodell (M) mit einer Anzahl von morphologisch ähnlichen Zahnmodellen (M) desselben Zahntyps verlinkt (L) ist,
- für jeden gewünschten Zahntyp ein optimales Zahnmodell (M) aus der Modelldatenbank (DB) durch ein iteratives Verfahren bestimmt wird, bei dem zunächst zumindest ein Start-Zahnmodell (M) des gewünschten Zahntyps aus der Modelldatenbank (DB) ausgewählt und dann beginnend mit diesem Start-Zahnmodell (M) bei jedem Iterationsschritt (S) ein Zahnmodell (M) hinsichtlich eines Qualitätswerts getestet wird, wobei
- das aktuell zu testende Zahnmodell (M) zur Individualisierung durch Variation von Modellparametern an die Scandaten (D) angepasst und ein Qualitätswert für diese Individualisierung berechnet wird,
- zumindest ein mit dem zu testenden Zahnmodell (M) verlinktes Zahnmodell (M) ebenfalls zur Individualisierung durch Variation von Modellparametern an die Scandaten (D) angepasst und ein weiterer Qualitätswert für diese Individualisierung berechnet wird,
- auf Basis der berechneten Qualitätswerte gegebenenfalls ein neues zu testendes Zahnmodell (M) des gewünschten Zahntyps aus der Modelldatenbank (DB) für den nächsten Iterationsschritt (S) ausgewählt wird,
- die Iteration bei Erreichung eines Qualitätskriteriums abgebrochen wird,
- und schließlich aus den optimalen Zahnmodellen (M) und Scandaten (D) zumindest eine virtuelle Zahnrestauration (R) bestimmt wird.

2. Verfahren nach Anspruch 1, **dadurch gekennzeichnet, dass** für jeden gewünschten Zahntyp einer Gruppe von Zahntypen ein optimales Zahnmodell (M) aus der Modelldatenbank (DB) durch ein iteratives Verfahren bestimmt wird, bei dem zunächst für jeden gewünschten Zahntyp zumindest ein Start-Zahnmodell (M) aus der Modelldatenbank (DB) ausgewählt und dann beginnend mit diesen Start-Zahnmodellen (M) bei jedem Iterationsschritt (S) eine Gruppe von Zahnmodellen (M) hinsichtlich eines (Gruppen-)Qualitätswerts getestet wird, wobei
- die aktuell zu testende Gruppe von Zahnmodellen (M) zur Individualisierung durch Variation von Modellparametern an die Scandaten (D) angepasst und ein Qualitätswert für diese Individualisierung berechnet wird,
- zumindest eine weitere Gruppe von Zahnmodellen (M) ebenfalls zur Individualisierung durch Variation von Modellparametern an die Scandaten (D) angepasst und ein weiterer Qualitätswert berechnet wird, wobei zur Bildung der weiteren Gruppe zumindest ein Zahnmodell (M) der aktuell zu testenden Gruppe durch ein mit ihm verlinktes Zahnmodell (M) ersetzt wird,
- auf Basis der berechneten Qualitätswerte gegebenenfalls eine neue zu testende Gruppe von Zahnmodellen (M) der gewünschten Zahntypen aus der Modelldatenbank (DB) für den nächsten Iterationsschritt (S) ausgewählt wird,
- die Iteration bei Erreichung eines Qualitätskriteriums abgebrochen wird,

3. Verfahren nach einem der Ansprüche 1 bis 2, **dadurch gekennzeichnet, dass** die Individualisierung von Zahnmodellen (M) durch die Lösung einer Optimierungsaufgabe durchgeführt wird, bei der sich der Optimierungswert aus einer Anzahl von Optimierungsteilwerten ergibt, wobei zumindest einzelne der Optimierungsteilwerte so gewählt sind, dass sie zumindest eines der folgenden Optimierungskriterien beschreiben:
- die Anpassung von Zahnmodellen (M) an die Zähne und/oder Restzahnsubstanzen,
- die Anpassung von Zahnmodellen (M) an die Gegenbezahnung,
- die Anpassung von Zahnmodellen (M) an Bissregistrate,
- die Anpassung von Zahnmodellen (M) an Nachbarzähne,
- die Anpassung von Zahnmodellen (M) an Präparationslinien (LP) und/oder Segmentierungslinien (LS),
- die Anpassung von Zahnmodellen (M) an anatomische Landmarken (AL),
- die mechanische Stabilität der zu Zahnmodellen (M) gehörenden virtuellen Zahnrestaurationen (R),
- die ästhetische Wirkung der zu Zahnmodellen (M) gehörenden virtuellen Zahnrestaurationen (R),
- die Kontakte von Zahnmodellen (M),
- die räumlichen Relationen der Lagen von Zahnmodellen (M),
- die räumlichen Relationen der Formen von Zahnmodellen (M).

4. Verfahren nach einem der Ansprüche 1 bis 3, **dadurch gekennzeichnet, dass** nach Bestimmung der optimalen Zahnmodelle (M) und vor Bestimmung der virtuellen Zahnrestaurationen (R) eine Feinanpassung der optimalen Zahnmodelle (M) untereinander und/oder an die Scandaten (D) stattfindet.

5. Verfahren nach einem der Ansprüche 1 bis 4, **dadurch gekennzeichnet, dass** die Suche nach einem optimalen Zahnmodell (M) eines Zahntyps bei zumindest einem Start-Zahnmodell (M) beginnt,
- das in der Modelldatenbank (DB) festgelegt ist und/oder
- das ein Mittelwert-Zahnmodell (M) von Zahnmodellen (M) des Zahntyps ist und/oder
- das aus einer geometrischen Analyse der Scandaten (D) bestimmt wird.

6. Verfahren nach einem der Ansprüche 1 bis 5, **dadurch gekennzeichnet, dass** die Suche nach einem optimalen Zahnmodell (M) eines Zahntyps bei mehreren Start-Zahnmodellen (M) des Zahntyps beginnt,
- die in der Modelldatenbank (DB) festgelegt sind und/oder
- die Mittelwert-Zahnmodelle (M) von Untergruppen der Zahnmodelle (M) des Zahntyps sind und/oder
- die aus einer geometrischen Analyse der Scandaten (D) bestimmt werden.

7. Verfahren nach einem der Ansprüche 1 bis 6, **dadurch gekennzeichnet, dass** die vorhandenen Zahntypen einer Modelldatenbank (DB) zur Auswahl einer Auswahleinheit zugeführt werden und mit Hilfe eines Auswahlsignals gewünschte Zahntypen selektiert und das Verfahren auf Basis der so ausgewählten Zahntypen durchgeführt wird.

8. Verfahren nach einem der Ansprüche 1 bis 7, **dadurch gekennzeichnet, dass** in der Modelldatenbank (DB) vorhandene Zahntypen und/oder Zahnmodelle (M) eines gewünschten Zahntyps zur Auswahl einer Auswahleinheit zugeführt werden und mit Hilfe eines Auswahlsignals gewünschte Zahntypen und/oder Zahnmodelle (M) selektiert und auf dieser Basis die optimalen Zahnmodelle (M) bestimmt werden.

9. Verfahren nach einem der Ansprüche 1 bis 8, **dadurch gekennzeichnet, dass** die Formparameter der Zahnmodelle (M) entsprechend ihres Einflusses auf die Zahnmodellgeometrie geordnet sind und/oder dass die Formparameter der Zahnmodelle (M) dreidimensionale Transformationsfelder für die Zahnmodelle (M) parametrisieren und/oder geometrische Konstruktionsparameter sind.

10. Verfahren zur Herstellung oder Auswahl eines Zahnrestaurationsteils, wobei zunächst auf der Basis von Scandaten (D) oraler Strukturen eine virtuelle Zahnrestauration (R) mittels eines Verfahrens nach einem der Ansprüche 1 bis 9 ermittelt wird und dann basierend auf der ermittelten virtuellen Zahnrestauration (R) das Zahnrestaurationsteil hergestellt oder aus einer Menge von vorgefertigten Zahnrestaurationsteilen ausgewählt wird.

11. Verfahren zur Erzeugung einer Modelldatenbank (DB), die für verschiedene Zahntypen jeweils eine Anzahl von parametrisierten Zahnmodellen (M) enthält, zur Verwendung in einem Verfahren nach einem der Ansprüche 1 bis 9, wobei die Parametrisierung anhand von Modellparametern erfolgt, welche Lage- und/oder Formparameter umfassen und wobei jedes Zahnmodell (M) mit einer Anzahl von morphologisch ähnlichen Zahnmodellen (M) desselben Zahntyps verlinkt (L) wird.

12. Verfahren nach Anspruch 11, **dadurch gekennzeichnet, dass** zumindest Teile der Modelldatenbank (DB) durch die Analyse einer Menge von Scandaten (D) künstlicher und/oder natürlicher oraler Strukturen aufgebaut werden, bei dem für einen gewünschten Zahntyp
- die Scandaten (D) optional segmentiert werden, um Scandaten (D) des gewünschten Zahntyps zu erhalten,
- Zahnmodelle (M) der Modelldatenbank (DB) durch eine Anpassung an die Scandaten (D) des gewünschten Zahntyps konstruiert werden,
- eine Analyse von morphologischen Abweichungen der Zahnmodelle (M) untereinander durchgeführt wird, indem für jedes mögliche Paar von Zahnmodellen (M) ein Abweichungswert berechnet wird,
- Cluster (C₁, C₂, C₃, C₄) von morphologisch ähnlichen Zahnmodellen (M) durch eine Analyse der Abweichungswerte der Zahnmodelle (M) untereinander gebildet werden,
- eine Verlinkung (L) von Zahnmodellen (M) innerhalb der Cluster (C₁, C₂, C₃, C₄) und von Mittelwert-Zahnmodellen (M) der Cluster (C₁, C₂, C₃, C₄) untereinander stattfindet.

13. Verfahren nach Anspruch 12, **dadurch gekennzeichnet, dass** parametrisierte geometrische Transformationen zu den Zahnmodellen (M) hinzugefügt werden, dadurch definiert, dass die Form eines Zahnmodells (M) in zumindest ein mit ihm verlinktes Zahnmodells (M) des gleichen Clusters (C₁, C₂, C₃, C₄) kontinuierlich überführt werden kann.

14. Computerprogrammprodukt, welches direkt in einen Speicher eines Computers ladbar ist, mit Programmcode-Mitteln, um alle Schritte eines Verfahrens nach einem der Ansprüche 1 bis 13 auszuführen, wenn das Programmprodukt auf dem Computer ausgeführt wird.

15. Zahnrestaurationsermittlungssystem (5) zur Ermittlung von virtuellen Zahnrestaurationen auf der Basis von Scandaten (D) oraler Strukturen, mit
- einer Schnittstelle (11) zum Empfang der von einer Messeinrichtung gemessenen Scandaten (D),
- einer Auswahleinheit (14), um die vom Verfahren zu verwendenden Zahntypen festzulegen,
- einer Speichereinrichtung (12) mit einer Modelldatenbank (DB), die für verschiedene Zahntypen jeweils eine Anzahl von parametrisierten Zahnmodellen (M) enthält, wobei die Parametrisierung anhand von Modellparametern erfolgt, welche Lage- und/oder Formparameter umfassen, und wobei jedes Zahnmodell (M) mit einer Anzahl von morphologisch ähnlicher Zahnmodellen (M) desselben Zahntyps verlinkt (L) ist,
- einer Optimierungseinheit (15), welche ausgebildet ist, um für jeden gewünschten Zahntyp ein optimales Zahnmodell (M) aus der Modelldatenbank (DB) durch ein iteratives Verfahren zu bestimmen, bei dem beginnend mit zumindest einem Start-Zahnmodell (M) bei jedem Iterationsschritt (S) ein Zahnmodell (M) hinsichtlich eines Qualitätswerts getestet wird,
- einer Ladeeinheit (16), welche ausgebildet ist, um Zahnmodelle (M) aus einer Modelldatenbank (DB) zu laden,
- einer Individualisierungseinheit (17), welche ausgebildet ist, um zur Individualisierung zumindest ein aktuell zu testendes Zahnmodell (M) und zumindest ein mit dem zu testenden Zahnmodell (M) verlinktes Zahnmodell (M) durch Variation von Modellparametern an die Scandaten (D) anzupassen,
- einer Qualitätsermittlungseinheit (18), welche ausgebildet ist, um Qualitätswerte für die Individualisierung der Zahnmodelle (M) zu ermitteln und Qualitätskriterien für den Abbruch der Iteration zu überprüfen,
- und einer Restaurationseinheit (19), welche ausgebildet ist, um aus den optimalen Zahnmodellen (M) und Scandaten (D) zumindest eine virtuelle Zahnrestauration (R) zu bestimmen.

## Claims

1. Method of determining virtual tooth restorations on the basis of scan data (D) of oral structures, wherein
- a model database (DB) comprising a number of parameterized tooth models (M) for each of several tooth types is used, whereby the parameterization is carried out on the basis of model parameters comprising position parameters and/or shape parameters and whereby each tooth model (M) is linked (L) with a number of morphologically similar tooth models (M) of the same tooth type,
- for each desired tooth type, an optimal tooth model (M) in the model database (DB) is determined by means of an iterative method in which initially at least one start tooth model (M) of the desired tooth type is selected from the model database (DB), and subsequently, commencing with this start tooth model (M), in each iteration step (S) a tooth model (M) is tested with regard to a quality value, wherein
- for individualization, the tooth model (M) currently in test is adjusted to the scan data (D) by varying model parameters and a quality value is computed for this individualization,
- at least one tooth model (M) linked with the tooth model (M) in test is, also for individualization, adjusted to the scan data (D) by variation of model parameters, and a further quality value is computed for this individualization,
- on the basis of the computed quality values, a new tooth model (M) in test of the desired tooth type is selected if necessary from the model database (DB) for the next iteration step (S),
- iteration is interrupted upon reaching a quality criterion,
- and finally at least one virtual tooth restoration (R) is determined from among the optimal tooth models (M) and scan data (D).

2. Method according to claim 1, **characterized in that**, for each desired tooth type of a tooth type group, an optimal tooth model (M) in the model database (DB) is determined by an iterative method in which initially, for each desired tooth type, at least one start tooth model (M) is selected from the model database (DB) and then, commencing with these start tooth models (M), a group of tooth models (M) is tested in each iteration step (S) with regard to a (group) quality value,
wherein
- the tooth model (M) group currently in test is adjusted for individualization to the scan data (D) by variation of model parameters and a quality value is computed,
- at least one further group of tooth models (M) is also adjusted for individualization to the scan data (D) by variation of model parameters, and a further quality value is computed, whereby, to form the further group, at least one tooth model (M) of the group currently in test is replaced by a tooth model (M) that is linked to it,
- if necessary, a new group of tooth models (M) of the desired tooth types is selected for the next iteration step (S) from the model database (DB), on the basis of the computed quality values,
- the iteration is interrupted upon reaching a quality criterion.

3. Method according to any of claims 1 to 2, **characterized in that** the individualization of tooth models (M) is performed by solving an optimization problem, in which an optimization value results from a number of optimization partial values, whereby at least some of the optimization partial values are chosen to describe at least one of the following optimization criteria:
- the adjustment of tooth models (M) to teeth and/or remaining tooth structure,
- the adjustment of tooth models (M) to opposing dentition,
- the adjustment of tooth models (M) to bite registrations,
- the adjustment of tooth models (M) to adjacent teeth,
- the adjustment of tooth models (M) to preparation lines (LP) and/or segmentation lines (LS),
- the adjustment of tooth models (M) to anatomical landmarks (AL),
- the mechanical stability of virtual tooth restorations (R) belonging to tooth models (M),
- the aesthetic effect of virtual tooth restorations (R) belonging to tooth models (M),
- the contacts of tooth models (M),
- the spatial relations of the positions of tooth models (M)
- the spatial relations of the shapes of tooth models (M).

4. Method according to any of claims 1 to 3, **characterized in that**, after determination of the optimal tooth models (M) and prior to determining the virtual tooth restorations (R), a precision adjustment of the optimal tooth models (M) is performed relative to each other and/or to the scan data (D).

5. Method according to any of claims 1 to 4, **characterized in that** the search for an optimal tooth model (M) of a tooth type commences with at least one start tooth model (M), which start tooth model (M)
- is defined in the model database (DB) and/or
- is a mean tooth model (M) of tooth models (M) of the tooth type and/or
- is determined from a geometrical analysis of the scan data.

6. Method according to any of claims 1 to 5, **characterized in that** the search for an optimal tooth model (M) of a tooth type commences with several start tooth models (M) of the tooth type, which start tooth models (M)
- are defined in the model database (DB) and/or
- are mean tooth models (M) of sub-groups of the tooth models (M) of the tooth type and/or
- are determined from a geometrical analysis of the scan data.

7. Method according to any of claims 1 to 6, **characterized in that** the available tooth types of a model database (DB) are forwarded for selection to a selection unit, and desired tooth types are selected with the aid of a selection signal, and the method is performed on the basis of the tooth types thus selected.

8. Method according to any of claims 1 to 7, **characterized in that** tooth types and/or tooth models (M) of a desired tooth type, available in the model database (DB), are forwarded for selection to a selection unit, and desired tooth types and/or tooth models (M) are selected with the aid of a selection signal, and the optimal tooth models (M) are determined on this basis.

9. Method according to any of claims 1 to 8, **characterized in that** the shape parameters of the tooth models (M) are ordered according to their influence on the tooth model geometry and/or **in that** the shape parameters of the tooth models (M) parameterize three-dimensional transformation fields for the tooth models (M) and/or are geometrical construction parameters.

10. Method of manufacturing or selecting a tooth restoration part, wherein at first, on the basis of scan data (D) of oral structures, a virtual tooth restoration (R) is determined by a method according to any of claims 1 to 9, and the tooth restoration part is subsequently manufactured or chosen from a set of prefabricated tooth restoration parts, based on the determined virtual tooth restoration (R).

11. Method of generating a model database (DB) comprising a number of parameterized tooth models (M) for each of a number of different tooth types, for use in the method according to any of claims 1 to 9, whereby parameterization is performed on the basis of model parameters comprising position parameters and/or shape parameters, and whereby each tooth model (M) is linked (L) with a number of morphologically similar tooth models (M) of the same tooth type.

12. Method according to claim 11, **characterized in that** at least parts of the model database (DB) are built by the analysis of a set of scan data (D) of artificial and/or natural oral structures, in which, for a desired tooth type,
- the scan data (D) are optionally segmented in order to obtain scan data (D) of the desired tooth type,
- tooth models (M) of the model database (DB) are constructed by adjustment to the scan data (D) of the desired tooth type,
- an analysis of morphological differences among the tooth models is carried out, wherein a difference value is computed for each possible pair of tooth models (M),
- clusters (C₁, C₂, C₃, C₄) of morphologically similar tooth models (M) are formed by an analysis of difference values amongst the tooth models (M),
- a linking (L) of tooth models (M) within the clusters (C₁, C₂, C₃, C₄) and of mean tooth models (M) of the clusters (C₁, C₂, C₃, C₄) is performed.

13. Method according to claim 12, **characterized in that** parameterized geometrical transformations are added to the tooth models (M), defined **in that** the shape of a tooth model (M) can be smoothly transformed to at least one tooth model (M) of the same cluster (C₁, C₂, C₃, C₄) linked to that tooth model (M).

14. A computer program product, directly loadable in the memory of a computer, comprising program code means for carrying out all steps of a method according to any of claims 1 to 13 when said computer program product is run on the computer.

15. Tooth restoration determination system (5) for determining virtual tooth restorations on the basis of scan data (D) of oral structures, comprising
- an interface (11) for receiving scan data (D) measured by a measurement means,
- a selection unit (14) for determining the tooth types to be used by the method,
- a memory means (12) with a model database (DB) that comprises a number of parameterized tooth models (M) for each of several tooth types, wherein the parameterization is performed using model parameters comprising position parameters and/or shape parameters, and wherein each tooth model (M) is linked (L) with a number of morphologically similar tooth models (M) of the same tooth type,
- an optimization unit (15), realised to determine an optimal tooth model (M) from the model database (DB) for each desired tooth type, using an iterative method in which, commencing with at least one start tooth model (M) a tooth model (M) is tested with regard to a quality value at each iteration step (S),
- a loading unit (16), realised to load tooth models (M) from a model database (DB),
- an individualization unit (17), realised to adjust at least one tooth model (M) currently in test, and at least one tooth model (M) linked to the tooth model (M) currently in test, to the scan data (D) for individualization by varying model parameters,
- a quality determination unit (18), realised to determine quality values for individualization of the tooth models (M) and to assess quality criteria for interruption of the iteration, and
- a restoration unit (19), realised to determine at least one virtual tooth restoration (R) from the optimal tooth models (M) and scan data (D).

## Revendications

1. Procédé pour la détermination de restaurations dentaires virtuelles sur la base de données de balayage (D) de structures orales, dans lequel
- on utilise une base de données de modèles (DB) qui contient, pour différents types de dents, respectivement un certain nombre de modèles de dents (M) paramétrés, le paramétrage s'effectuant au moyen de paramètres de modèles comprenant des paramètres de position et/ou de forme, et chaque modèle de dent (M) étant lié (L) à un certain nombre de modèles de dents (M) morphologiquement similaires du même type de dent,
- pour chaque type de dent souhaité, un modèle de dent (M) optimal de la base de donnée de modèles (DB) étant déterminé grâce à un procédé itératif dans lequel on sélectionne tout d'abord au moins un modèle de dent (M) de départ du type de dent souhaité dans la base de données de modèles (DB) et ensuite, en commençant avec ce modèle de dent de départ (M), à chaque étape d'itération (S), un modèle de dent (M) étant testé concernant une valeur de qualité, dans lequel
- le modèle de dent (M) à tester actuellement est adapté aux données de balayage (D) pour l'individualisation grâce à la variation de paramètres de modèles, et une valeur de qualité pour cette individualisation étant calculée,
- au moins un modèle de dent (M) lié au modèle de dent (M) à tester étant également adapté aux données de balayage (D) pour l'individualisation grâce à la variation de paramètres de modèles, et une autre valeur de qualité étant calculée pour cette individualisation,
- sur la base des valeurs de qualité calculées, le cas échéant, un nouveau modèle de dent (M) à tester du type de dent souhaité de la base de données de modèles (DB) étant sélectionné pour la prochaine étape d'itération(S),
- l'itération étant interrompue lorsqu'un critère de qualité est atteint,
- et finalement, au moins une restauration dentaire virtuelle (R) étant déterminée à partir des modèles de dent optimaux (M) et des données de balayage (D).

2. Procédé selon la revendication 1, **caractérisé en ce que**, pour chaque type de dent souhaité d'un groupe de types de dents, on détermine un modèle de dent (M) optimal à partir de la base de données de modèles (DB) grâce à un procédé itératif dans lequel on sélectionne tout d'abord, pour chaque type de dent souhaité, au moins un modèle de dent (M) de départ dans la base de données de modèles (DB) et ensuite, en débutant avec ces modèles de dents de départ (M), à chaque étape d'itération (S), un groupe de modèles de dents (M) étant testé concernant une valeur de qualité (de groupe), dans lequel
- le groupe de modèles de dents (M) à tester actuellement étant adapté aux données de balayage (D) pour l'individualisation grâce à la variation de paramètres de modèles, et une valeur de qualité étant calculée pour cette individualisation,
- au moins un autre groupe de modèles de dents (M) étant également adapté aux données de balayage (D) pour l'individualisation grâce à la variation de paramètres de modèles et une autre valeur de qualité étant calculée, dans lequel, pour la formation de l'autre groupe, au moins un modèle de dent (M) du groupe à tester actuellement est remplacé par un modèle de dent (M) lié à lui,
- sur la base des valeurs de qualité calculées, le cas échéant, un nouveau groupe de modèles de dents (M) à tester des types de dents souhaités étant sélectionné dans la base de données de modèles (DB) pour la prochaine étape d'itération (S),
- l'itération étant interrompue lorsqu'un critère de qualité est atteint,

3. Procédé selon l'une des revendications 1 à 2, **caractérisé en ce que** l'individualisation de modèles de dents (M) est réalisée par résolution d'une tâche d'optimisation où la valeur d'optimisation résulte d'un certain nombre de valeurs partielles d'optimisation, dans lequel au moins certaines des valeurs partielles d'optimisation sont sélectionnées de manière à ce qu'elles décrivent au moins l'un des critères d'optimisation suivants :
- l'adaptation de modèles de dents (M) aux dents et/ou substances de restauration,
- l'adaptation de modèles de dents (M) à la denture opposée,
- l'adaptation de modèles de dents (M) à des empreintes de l'occlusion,
- l'adaptation de modèles de dents (M) à des dents voisines,
- l'adaptation de modèles de dents (M) à des lignes de préparation (LP) et/ou des lignes de segmentation (LS),
- l'adaptation de modèles de dents (M) à des points de repère anatomiques (AL),
- la stabilité mécanique des restaurations dentaires virtuelles (R) faisant partie de modèles de dents (M),
- l'effet esthétique des restaurations dentaires virtuelles (R) faisant partie de modèles de dents (M),
- les contacts de modèles de dents (M),
- les relations spatiales des positions de modèles de dents (M),
- les relations spatiales des formes des modèles de dents (M).

4. Procédé selon l'une des revendications 1 à 3, **caractérisé en ce que**, après la détermination des modèles de dents (M) optimaux et avant la détermination des restaurations dentaires virtuelles (R), il y a une adaptation de précision des modèles de dents (M) optimaux entre eux et/ou aux données de balayage (D).

5. Procédé selon l'une des revendications 1 à 4, **caractérisé en ce que** la recherche d'un modèle de dent (M) optimal d'un type de dent débute avec au moins un modèle de dent de départ (M),
- lequel est fixé dans la base de données de modèles (DB) et/ou
- lequel est un modèle de dent (M) de valeur moyenne de modèles de dents (M) du type de dents et/ou
- lequel est déterminé à partir d'une analyse géométrique des données de balayage (D).

6. Procédé selon l'une des revendications 1 à 5, **caractérisé en ce que** la recherche d'un modèle de dent (M) optimal d'un type de dents débute avec plusieurs modèles de dent de départ (M) du type de dent,
- lesquels sont déterminés dans la base de données de modèles (DB) et/ou
- lesquels sont des modèles de dents de valeur moyenne (M) de sous-groupes des modèles de dents (M) du type de dent et/ou
- lesquels sont déterminés à partir d'une analyse géométrique des données de balayage (D).

7. Procédé selon l'une des revendications 1 à 6, **caractérisé en ce que** les types de dents existants d'une base de données de modèles (DB) sont amenés pour la sélection à une unité de sélection et **en ce qu'**à l'aide d'un signal de sélection, des types de dent souhaités sont sélectionnés et le procédé étant mis en oeuvre sur la base des types de dents ainsi sélectionnés.

8. Procédé selon l'une des revendications 1 à 7, **caractérisé en ce que** des types de dents existant dans la base de données de modèles (DB) et/ou des modèles de dents (M) d'un type de dent souhaité sont amenés pour la sélection à une unité de sélection et **en ce qu'**à l'aide d'un signal de sélection, des types de dent souhaités et/ou modèles de dents (M) sont sélectionnés, et les modèles de dents (M) optimaux étant sélectionnés sur cette base.

9. Procédé selon l'une des revendications 1 à 8, **caractérisé en ce que** les paramètres de forme des modèles de dent (M) sont classés en fonction de leur influence sur la géométrie du modèle de dent et/ou **en ce que** les paramètres de forme des modèles de dent (M) assurent le paramétrage de champs de transformation tridimensionnels pour les modèles de dents (M) et/ou sont des paramètres de construction géométriques.

10. Procédé pour la fabrication ou la sélection d'un élément de restauration dentaire, dans lequel on détermine tout d'abord, sur la base de données de balayage (D) de structures orales, une restauration dentaire virtuelle (R) au moyen d'un procédé selon l'une des revendications 1 à 9, et ensuite, sur la base de la restauration dentaire virtuelle (R) déterminée, l'élément de restauration dentaire étant fabriqué ou sélectionné à partir d'une quantité d'éléments de restauration dentaire préfabriqués.

11. Procédé pour la création d'une base de données de modèles (DB), laquelle contient, pour différents types de dents, respectivement un certain nombre de modèles de dents (M) paramétrés pour l'utilisation dans un procédé selon l'une des revendications 1 à 9, dans lequel le paramétrage s'effectue à l'aide de paramètres de modèles qui comprennent des paramètres de position et/ou de forme et dans lequel chaque modèle de dent (M) est lié à un certain nombre de modèles de dents (M) morphologiquement similaires du même type de dent.

12. Procédé selon la revendication 11, **caractérisé en ce qu'**au moins des parties de la base de données de modèles (DB) sont élaborées grâce à l'analyse d'une quantité de données de balayage (D) de structures orales artificielles et/ou naturelles, dans lequel, pour un type de dent souhaité
- les données de balayage (D) sont optionnellement segmentées afin d'obtenir des données de balayage (D) du type de dent souhaité,
- des modèles de dent (M) de la base de données de modèles (DB) étant construits grâce à une adaptation aux données de balayage (D) du type de dent souhaité,
- une analyse de divergences morphologiques des modèles de dents (M) entre eux étant effectuée **en ce que**, pour chaque paire possible de modèles de dents (M), une valeur de divergence est calculée,
- des regroupements (C₁, C₂, C₃, C₄) de modèles de dents (M) morphologiquement similaires étant formés par analyse des valeurs de divergence des modèles de dents (M) entre eux,
- un établissement de lien (L) de modèles de dents (M) au sein des regroupements (C₁, C₂, C₃, C₄) et de modèles de dents de valeur moyenne (M) des regroupements (C₁, C₂, C₃, C₄) ayant lieu entre eux.

13. Procédé selon la revendication 12, **caractérisé en ce que** des transformations géométriques paramétrées sont ajoutées aux modèles de dents (M), défini **en ce que** l'on peut faire passer la forme d'un modèle de dent (M) à au moins un modèle de dent (M) lié à lui du même regroupement (C₁, C₂, C₃, C₄) de manière continue.

14. Produit de programme informatique, lequel peut être directement chargé dans une mémoire d'un ordinateur, avec des moyens de code de programme afin d'exécuter toutes les étapes d'un procédé selon l'une des revendications 1 à 13 lorsque le produit de programme est exécuté sur l'ordinateur.

15. Système de détermination de restauration dentaire (5) pour déterminer des restaurations dentaires virtuelles sur la base de données de balayage (D) de structures orales, avec
- une interface (11) pour la réception des données de balayage (D) mesurées par un dispositif de mesure,
- une unité de sélection (14) afin de fixer les types de dent à utiliser par le procédé,
- un dispositif de mémoire (12) avec une base de données de modèles (DB) laquelle contient, pour différents types de dents, respectivement un certain nombre de modèles de dents (M) paramétrés, dans lequel le paramétrage s'effectue au moyen de paramètres de modèles comprenant des paramètres de position et/ou de forme, et dans lequel chaque modèle de dent (M) du même type de dent est lié (L) à un certain nombre de modèles de dents (M) morphologiquement semblables du même type de dent,
- une unité d'optimisation (15), laquelle est réalisée pour déterminer, pour chaque type de dent souhaité, un modèle de dent (M) optimal dans la base de données de modèles (DB) grâce à un procédé itératif, dans lequel, en débutant au moins avec un modèle de dent de départ (M), à chaque étape d'itération (S), un modèle de dent (M) est testé concernant une valeur de qualité,
- une unité de charge (16), laquelle est réalisée pour charger des modèles de dents (M) à partir d'une base de données de modèles (DB),
- une unité d'individualisation (17), laquelle est réalisée, pour l'individualisation, pour adapter aux données de balayage (D) au moins un modèle de dent (M) à tester actuel et au moins modèle de dent (M) lié au modèle de dent (M) à tester, grâce à la variation de paramètres de modèles,
- une unité de détermination de qualité (18), laquelle est réalisée pour déterminer des valeurs de qualité pour l'individualisation des modèles de dent (M) et pour vérifier des critères de qualité pour l'interruption de l'itération,
- et une unité de restauration (19), laquelle est réalisée pour déterminer au moins une restauration dentaire virtuelle (R) à partir des modèles de dent (M) optimaux et des données de balayage (D).
